# EUROPEAN PATENT SPECIFICATION

(11) **EP 0 651 783 B1**
(45) Date of publication and mention of the grant of the patent: **28.05.2003**
(21) Application number: 93915503.2
(22) Date of filing: 01.07.1993
(51) Int. Cl.: C12N 1/15, C12N 1/19, C12N 1/21, C12N 5/10, C12N 5/16, C12N 9/16, C12N 15/55, C12N 15/63, C12Q 1/34, C07H 21/04

(54) **ALKALINE PHOSPHATASE ENZYME HAVING IMPROVED SPECIFIC ACTIVITY FOR USE IN INDICATOR REAGENTS**
ALKALISCHE PHOSPHATASE-ENZYME MIT VERBESSERTER SPEZIFISCHER AKTIVITÄT IN DER VERWENDUNG ALS INDIKATOR-REAGENZIEN
ENZYME PHOSPHATASE ALCALINE AYANT UNE ACTIVITE SPECIFIQUE ACCRUE POUR UTILISATION DANS DES REACTIFS INDICATEURS.

(30) Priority: 07.07.1992 US 909915
(43) Date of publication of application: 10.05.1995
(73) Proprietor: ABBOTT LABORATORIES, Abbott Park, Illinois 60064-3500 (US)
(72) Inventor: MANDECKI, Wlodzimierz, Libertyville, IL 60048 (US); SHALLCROSS, Mary, Ann, Grayslake, IL 60030 (US); TOMAZIC-ALLEN, Susan, J., Grayslake, IL 60030 (US)
(74) Representative: Modiano, Guido, Dr.-Ing.
(86) International application number: US9306264
(87) International publication number: WO94001531

(56) References cited:
- EP-A- 0 441 252
- US-A- 4 804 625
- PROCEEDINGS OF THE NATIONAL ACADEMY OF SCIENCES OF USA, vol. 87, no. 2, 2 January 1990, pages 696-700, XP002016420 J.D. HERMES ET AL.: "Searching sequence space by definable random mutagenesis: Improving the catalytic potency of an enzyme."
- PROTEIN ENGINEERING, vol. 4, no. 7, October 1991, pages 801-804, XP002016421 W. MANDECKI ET AL.: "Mutagenesis of conserved residues within the active site of Escherichia coli alkaline phosphatase yields enzymes with increased k-cat."
- Protein Engineering, Volume 3, No. 2, issued 1989, A. CHAIDAROGLOU et al., "Alteration of Aspartate 101 in the Active Site of Escherichia Coli Alkaline Phosphatase Enhances the Catalytic Activity", pages 127-132, entire document.
- Journal of Molecular Biology, Volume 186, issued 1985, J.M. SOWADSKI et al., "Refined Structure of Alkaline Phosphatase from Escherichia Coli at 2.8 A Resolution", pages 417-433, entire document.
- Journal of Biological Chemistry, Volume 247, No. 7, issued 10 April 1972, S.E. HALFORD et al., "A Mutationally Altered Alkaline Phosphatase from Escherichia Coli", pages 2095-2101, entire document.
- Science, Volume 231, issued 10 January 1986, S.S. GHOSH et al., "Modification of the Active Site of Alkaline Phosphatase by Site-Directed Mutagenesis", pages 145-148, entire document.
- Biochemistry, Volume 27, No. 22, issued 1988, A. CHAIDAROGLOU et al., "Function of Arginine-166 in the Active Site of Escherichia Coli Alkaline Phosphatase", pages 8338-8343, entire document.
- Proceedings of the National Academy of Sciences, Vol. 85, issued June 1988, BUTLER-RANSONOFF et al., "Use of Site-Directed Mutagenesis to Elucidate the Role of Arginine-166 in the Catalytic Mechanism of Alkaline Phosphatase", pages 4276-4278, entire document.

## Description

### Field of the Invention

The present invention relates to the modification of enzymes to improve their biological properties. In particular, the invention relates to the use of a genetically engineered *Escherichia coli* (*E*. *coli*) to produce alkaline phosphatase for use as a label in binding assays, wherein the enzyme has an enhanced specific activity and a high thermal stability.

### Background of the Invention

Alkaline phosphatase is used as a readily detectable labeling enzyme in various diagnostic binding assays. For example, it is frequently used in heterogeneous enzyme binding assays wherein it may be coupled, depending upon the type of analysis, to an antigen, antibody or other specific binding member which will bind to the analyte of interest. After binding has occurred, the newly formed binding complex may be separated from the reaction mixture and detected by observing the presence or amount of alkaline phosphatase associated with the complex. The alkaline phosphatase is detected by adding an enzyme substrate and observing the extent of the resultant enzyme/substrate reaction.

The criteria for choosing any particular enzyme for use as a label include: a high specific activity (i.e., a high rate of catalysis or high velocity of the enzymatic reaction); stability at high temperatures (usually a melting temperature of greater than 50-60°C); stability of the enzyme after conjugation to the specific binding member, the availability of easily quantifiable enzyme substrates for use in the enzyme detection reaction; the availability of reaction product amplification methods; and suitable performance in the assay (e.g., low background readouts). Temperature stability is a major concern for many industrial applications of enzymes. Proteins vary with respect to their temperature stability, and the melting temperatures (Tₘ) of different enzymes can range from less than 40°C to greater than 100°C.

Calf-intestinal alkaline phosphatase is often used as an enzyme label, exhibiting both a high specific activity and a melting temperature of about 55°C. The enzyme and its conjugates are convenient to use in binding assays, and there are several methods of amplifying the product of the detection reaction. The use of calf-intestinal alkaline phosphatase as a label, however, still presents some disadvantages. These disadvantages include inadequate purity of some enzyme preparations and the presence of covalently linked carbohydrates which are believed to contribute to higher background readings when determining assay results. In addition, calf-intestinal alkaline phosphatase has a poor temperature stability after it is conjugated to a specific binding member for use in a binding assay. Because of these disadvantages, much research has been conducted involving the search for and development of a new form of alkaline phosphatase which has both a specific activity and a temperature stability more suitable for binding assays, especially those assays in which increased temperatures are reached.

One approach chosen to overcome the disadvantages of the mammalian enzyme relies upon the use of *E. coli* alkaline phosphatase which has an extremely high temperature stability in comparison with mammalian alkaline phosphatases. *E. coli* alkaline phosphatase has a Tₘ of about 95°C. Alkaline phosphatase can also be expressed in increased amounts from *E. coli*, if numerous copies of the corresponding gene per cell are present. In addition, the enzyme can be purified to homogeneity in a few steps. Alkaline phosphatase from *E. coli*, however, has a lower specific activity than does calf-intestinal alkaline phosphatase. The maximum velocity of the catalytic reaction (k_{cat}) for *E. coli* alkaline phosphatase is 60 sec⁻¹, although considerable variation has been observed. This compares with a k_{cat} of about 2,000 sec⁻¹ for calf-intestinal alkaline phosphatase. Because naturally occurring *E. coli* alkaline phosphatase has a lower rate of catalysis than does calf-intestinal alkaline phosphatase, it would be advantageous to improve the catalytic activity of the *E. coli* alkaline phosphatase enzyme while simultaneously conserving its more favorable temperature stability characteristic.

Recent progress in the field of protein engineering, such as site-directed mutagenesis, computer-assisted molecular design, gene expression technologies, and the availability of crystal or nuclear magnetic resonance structures, has made it feasible to undertake projects aimed at modifying a specific characteristic of an enzyme. The modifiable characteristics of an enzyme can be divided into two general classes: (1) those characteristics that depend upon the local properties of the enzyme's active site, such as substrate specificity, the velocity of the catalytic reaction (k_{cat}), the Michaelis constant (Kₘ), etc.; and (2) those characteristics that depend upon the global properties of the protein structure, such as temperature stability, resistance to proteolysis and allosteric regulation of the active site. The active site of an enzyme is typically composed of approximately ten amino acid residues. In general, the amino acids in the active site establish the processes that occur at the active site. Therefore, by changing the amino acid residues that compose the active site, the catalytic effects of the enzyme can be changed.

Early efforts to modify the properties of alkaline phosphatase relied mainly upon the chemical modification of the enzyme. For example, the enzyme was subjected to photo-oxidation, or to treatment with mono- and dichloroacetyl-β-glycerophosphate; 2,3-butanedione; phenylglyoxal and other compounds. Typically, these treatments reduced or eliminated the enzyme's catalytic activity, thereby providing information on the function of the enzyme [Coleman and Gettings, Adv. Enzymol., Volume 55, page 351 (1983)]. After the treatment of the enzyme with tetranitromethane (to nitrate the tyrosyl residues), followed by the reduction of the nitrated residues to aminotyrosyl, the enzyme's phosphohydrolase activity rose to 130% of the activity of the unmodified protein, whereas the phosphotransferase activity was 350% of the normal value [Christen, et al., Biochemistry, Volume 10, page 1377 (1971)]. Such experiments demonstrated that certain enzymatic properties of alkaline phosphatase could be improved by chemically modifying the enzyme. The chemical modification procedures, however, are limited in that they have a low selectivity as to which amino acids in the enzyme are modified, and thus, a broad spectrum of amino acid residues can be affected.

In addition, changes in the active or binding site of a protein can also be more extensive than the change of a single amino acid (i.e., point mutation). For example, proteins that repress gene expression (repressors) can be converted into activators by changing several of the acidic residues [Ma and Ptashne, Cell. Volume 48, pages 847-853 (1987)]. Most mutations reduce the Tₘ, as evidenced by the work on the alpha subunit of tryptophan synthetase (Yutant, et al., Nature. Volume 267, pages 274-275 (1977)]. Nevertheless, the replacement of a few amino acids in the enzyme's sequence can significantly increase the enzyme's Tₘ, as indicated by the results of the work on neutral protease of *Bacillus stearothermophilus* (Imanaka, et al., Nature, Volume 324, pages 695-697 (1986)].

The advent of recombinant DNA technology made it possible to modify or change a specific amino acid residue (site-directed mutagenesis) by modifying the gene which directs the formation of alkaline phosphatase (*phoA* gene). In *E. coli*, the *phoA* gene is part of the noncontinuous family of at least 18 genes which are involved in the regulation of phosphate binding, transport and metabolism. The nucleotide and amino acid sequences of the *E*. *coli phoA* gene are well-known to those skilled-in-the-art (Chang, Gene, Volume 44, pages 121-125 (1986)].

Through gene engineering, mutations of serine¹⁰² in the active site of the alkaline phosphatase enzyme resulted in a 1,000-fold reduction in specific activity, thereby demonstrating that this amino acid residue is critical for alkaline phosphatase activity [J.E. Butler-Ransohoff, et al., Abstracts of 1989 Alkaline Phosphatase Symposium, San Diego, CA, (1989)]. Alternatively, the substitution of serine with cysteine, which retains the hydroxy group, lead to only a slight reduction of activity [Ghosh, et al., Science, Volume 231, page 145 (1986)]. The role of arginine¹⁶⁶, a residue in the immediate vicinity of the enzyme's active site, was also investigated by mutagenesis, and resulted in an approximately 50-fold reduction in catalytic efficiency as compared to the native enzyme [Butler-Ransohoff, et al., Proc. Natl. Acad. Sci. USA., Volume 85, page 4276 (1988) and Chaidaroglou, et al., Biochemistry, Volume 27, page 8338 (1988)]. The proteolytic elimination of 10 to 40 amino acid residues from the amino terminus of alkaline phosphatase has also been shown to result in reduced temperature stability, while the specific activity of the enzyme remained unchanged or only slightly reduced [Chlebowski, et al., J. Biol. Chem., Volume 264, page 4523 (1989)]. Several silent mutations in the *phoA* gene have also been documented from isolates of wild-type *E. coli* [DuBose, et al., Proc. Natl. Acad. Sci. USA, Volume 85, page 7036 (1988)]. Most recently, the function of aspartic acid¹⁰¹ in the active site of *E. coli* alkaline phosphatase was investigated by site-specific mutagenesis, wherein that amino acid was replaced with alanine [Chaidaroglou, et al., Protein Engineering, Volume 3(2), pages 127-132 (1989)]. The mutant enzyme exhibited about a 3-fold higher activity than the wild-type enzyme, but the mutant enzyme also exhibited a substantial decrease in thermal stability.

EP-A-0 441 252 discloses a synthetic enzyme characterized by being an alkaline phosphatase enzyme produced by *Escherichia coli* wherein such alkaline phosphatase has at least one amino acid mutation and a resulting increased specific activity as compared to wild-type *Escherichia coli* alkaline phosphatase. Engineered DNA sequences used to produce the enzymes, plasmids containing the engineered DNA sequences, hosts containing the plasmids, and assays utilizing the enzymes in the form of indicator reagents are also described. In particular, EP-A-0 441 252 discloses examples of single amino acid changes which are the substitution of Val in place of Thr¹⁰⁰, Ile in place of Thr¹⁰⁰, Arg in place of Lys³²⁸, Ala in place of Val⁹⁹, Asp in place of Ala¹⁰³, Cys in place of Ala¹⁰³, Val in place of Thr¹⁰⁷ and Ser in place of Asp¹⁰¹. Examples of dual amino acid changes are Ala in place of Val⁹⁹ and Arg in place of Lys³²⁸, or Ala in place of Val³⁷⁷ and Gly in place of Ser⁴¹⁵.

### Summary of the Invention

The present invention involves the construction of synthetic genes which control the production of a mutant of an alkaline phosphatase enzyme produced by *Escherichia coli* having an enhanced catalytic activity an compared to the wild-type enzyme, characterized in that the mutation consists of Gly in place of Asp¹⁵³. The enzymatic activity of the enzyme of the present application has been increased as compared to the wild-type enzyme. Although the enzyme of the present application has a lower thermal stability as compared to the wild-type enzyme, the thermal stability is conserved to the extent that the enzyme is suitable for use in binding assays, i.e., the enzyme of the present application is not heat-inactivated under normal assay conditions. A enzyme, DNA sequences used to produce the enzyme, plasmids containing the engineered DNA sequences, hosts containing the plasmids, and assays utilizing the enzyme in the form of indicator reagents are described.

The present invention includes a DNA sequence, suitable for insertion within a vector, which involves a series of codons encoding an alkaline phosphatase enzyme, wherein the alkaline phosphatase has at least one amino acid mutation as compared to wild-type *E. coli* alkaline phosphatase, and wherein the alkaline phosphatase has an increased specific activity as compared to wild-type *E. coli* alkaline phosphatase, characterized in that the mutation consists of Gly in place of Asp¹⁵³. Plasmids containing such DNA sequence, as well as host cells containing such plasmids, are also described. Unicellular hosts are used and are typically selected from bacterial and fungal strains such as *E. coli, Bacillus, Streptomyces,* mammalian cells, yeast and other fungi. The choice is not critical to the present invention, although all hosts may not be equally efficient.

Furthermore, the present invention involves the use of such synthetic alkaline phosphatase enzyme as enzyme labels in binding assays. Indicator reagents, useful for determining the presence or amount of an analyte in a test sample, can be made by attaching a specific binding member directly or indirectly to the novel enzyme . The resultant indicator reagents are suitable for use in assay formats including, but not limited to, sandwich assays, competitive assays and direct and indirect assay formats.

### Brief Description of the Drawings

FIG. 1(a)-(c) illustrate synthetic oligonucleotides used to construct the alkaline phosphatase gene.
FIG. 2 illustrates the restriction map of plasmid pMA100.
FIG. 3(a)-(b) illustrates the nucleotide and amino acid sequences of the synthetic, wild-type *E. coli* alkaline phosphatase.
FIG. 4 illustrates the results of a binding assay for the detection of alpha-fetoprotein using a reference indicator reagent of EP-A-0 441 252.
FIG. 5 illustrates the results of a binding assay for the detection of a cancer antigen using a reference indicator reagent of EP-A-0 441 252.
FIG. 6 illustrates the time courses of irreversible thermoinactivation of conjugates containing calf-intestinal alkaline phosphatase and *E. coli* mutant alkaline phosphatase of EP-A-0 441 252.
FIG. 7 illustrates the nucleotide sequence of the synthetic plasmid pWM520.

The following symbols and abbreviations are used to denote base codes and amino acids:

### Base codes:

| Symbol | Nucleotide |
|---|---|
| A | adenosine |
| C | cytosine |
| G | guanine |
| T | thymine |

### Amino acid three-letter abbreviations:

| Abbreviation | Amino Acid Name | Abbreviation | Amino Acid Name |
|---|---|---|---|
| Ala | Alanine | Leu | Leucine |
| Arg | Arginine | Lys | Lysine |
| Asn | Asparagine | Met | Methionine |
| Asp | Aspartic Acid (Aspartate) | Phe | Phenylalanine |
| Cys | Cysteine | Pro | Proline |
| Gln | Glutamine | Ser | Serine |
| Glu | Glutamic Acid (Glutamate) | Thr | Threonine |
| Gly | Glycine | Trp | Trytophan |
| His | Histidine | Tyr | Tyrosine |
| Ile | isoleucine | Val | Valine |

### Detailed Description of the Invention

The present invention provides a alkaline phosphatase enzyme which has an enhanced specific activity, while the desirable temperature stability characteristic of the native enzyme has been conserved. The enzyme of the present invention is a mutant of an alkaline phosphatase enzyme produced by *Escherichia coli,* wherein said mutant alkaline phosphatase has an amino acid mutation as compared to wild-type *Escherichia coli* alkaline phosphatase consisting of Gly in place of Asp¹⁵³. For the purpose of understanding and carrying out the invention, disclosures relating to the above-mentioned genetically modified enzymes of EP-A-0 441252 are also included herein below as reference examples (see Table 1).

The genetically modified enzymes are more thermostable than calf-intestinal alkaline phosphatase, exhibiting a minimum half-life of five minutes at 75°C (pH 7.5). In addition, depending upon the assay conditions, the enzymatic activity of the enzymes has been increased 1.5- to 36-fold over that exhibited by the native enzyme. The largest increases in specific activity are observed when the enzymatic activities of the enzymes are measured in the presence of low concentrations of either Tris (0.05 M) or diethanolamine (0.05 M). The enhanced specific activity has been accomplished through a combination of molecular modeling, genetic engineering and site-directed mutagenesis.

Typically, the target sites for mutations in alkaline phosphatase were predetermined on the basis of the enzyme's crystal structure (Sowadski, et al., J. Mol. Biol., Volume186, pages 417-433 (1985). The criterion for the selection of a given amino acid as a target site was the amino acid's proximity to the active site of the enzyme molecule, in particular its proximity to the catalytic residue, Ser¹⁰².

To produce a defined amino acid change at a given site in the enzyme, an appropriate codon sequence encoding that amino acid was inserted into the *phoA* gene. Random amino acid changes were also accomplished by inserting the codon sequence NNN into the DNA sequence, where N is any of four nucleotides. One constructed mutant fortuitously carried two point mutations, i.e., two amino acid changes, which were believed to be a result of the chemical modification of the synthetic oligonucleotides used for gene synthesis.

### Construction Of Plasmids

Once the initial set of suitably mutated DNA molecules was obtained, two protein design strategies were used to produce mutant enzymes. First, different modified DNA subsequences were introduced into a single gene, and the resultant clones were screened for the production of mutant enzymes having a high specific activity. Second, a mutant DNA molecule was used as a genetic background for a second round of mutagenesis.

The first strategy involved designing and constructing a totally synthetic gene that encoded alkaline phosphatase. The synthetic *phoA* gene was synthesized using the *Fok*l method as described in Mandecki and Bolling, Gene, Volume 68, pages 101-107 (1988).

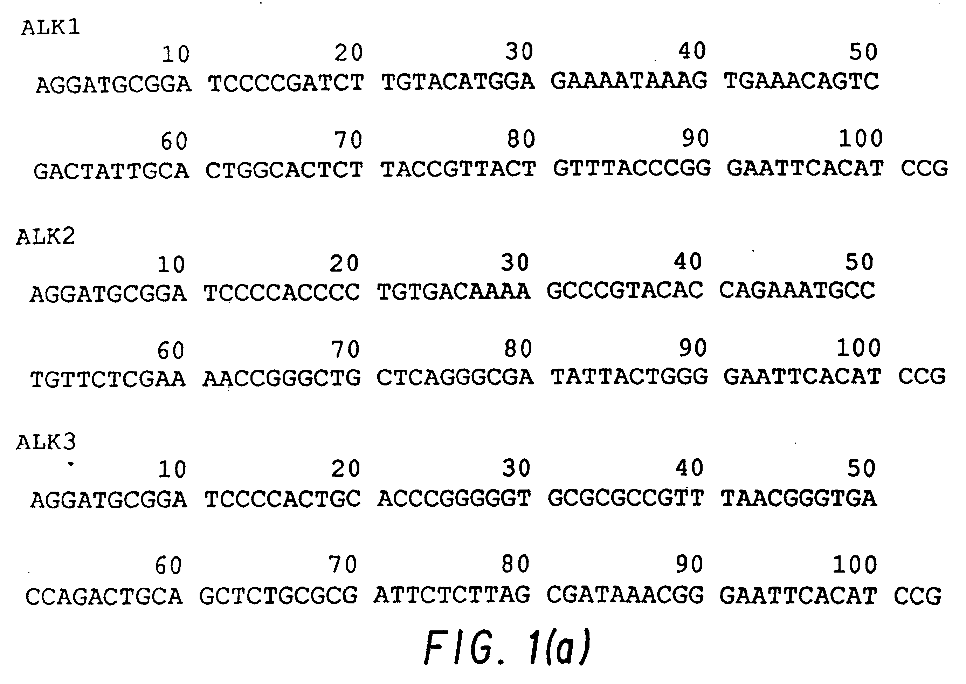

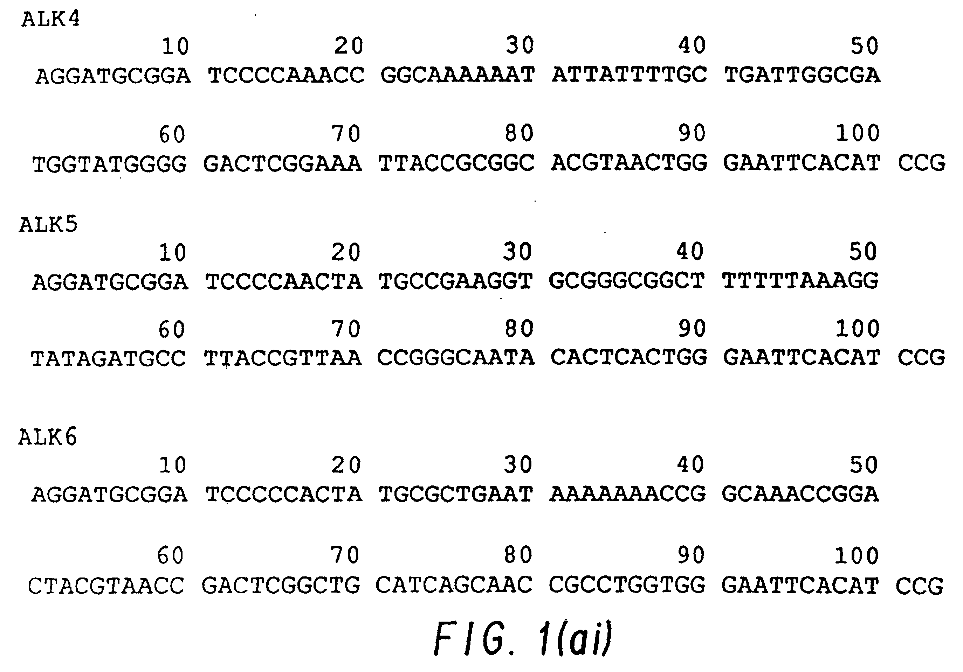

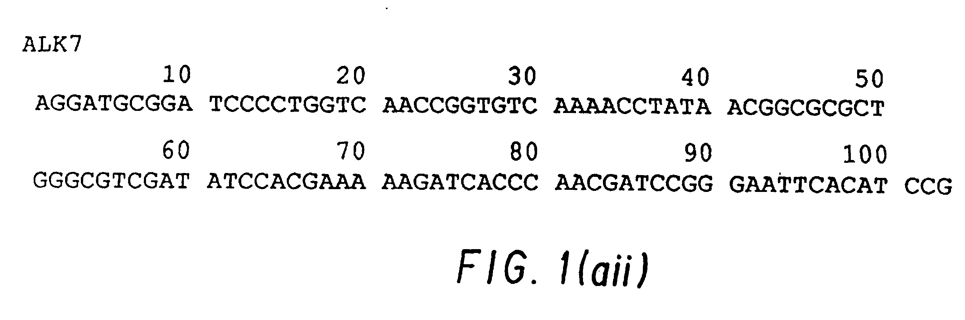

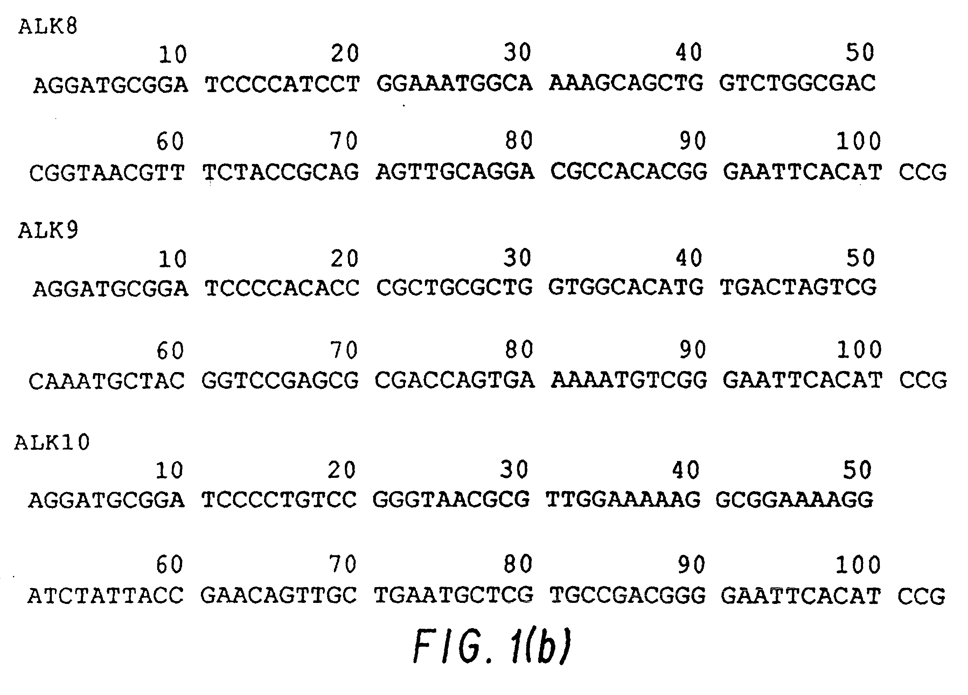

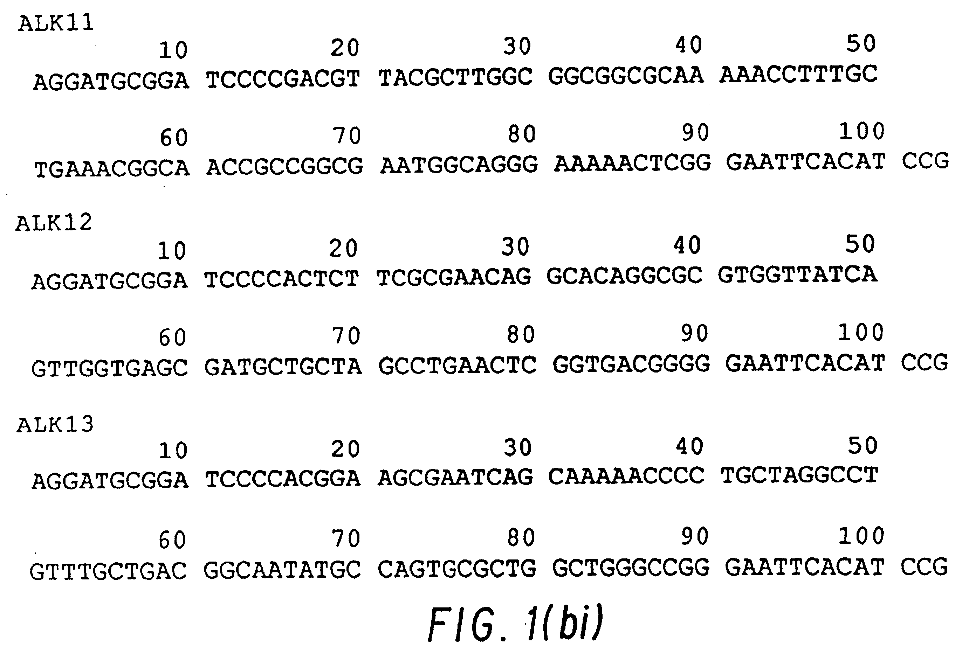

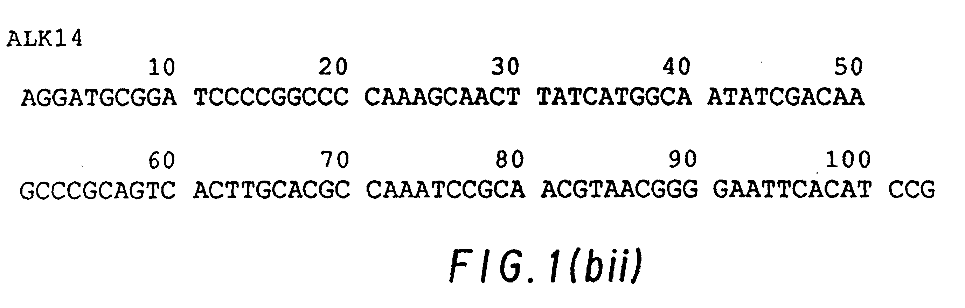

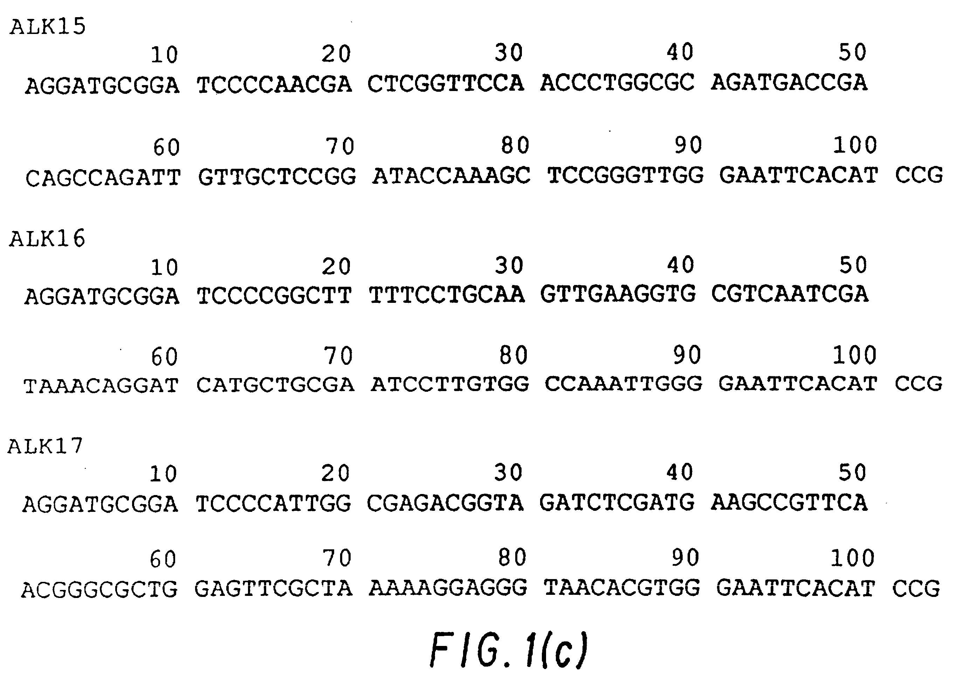

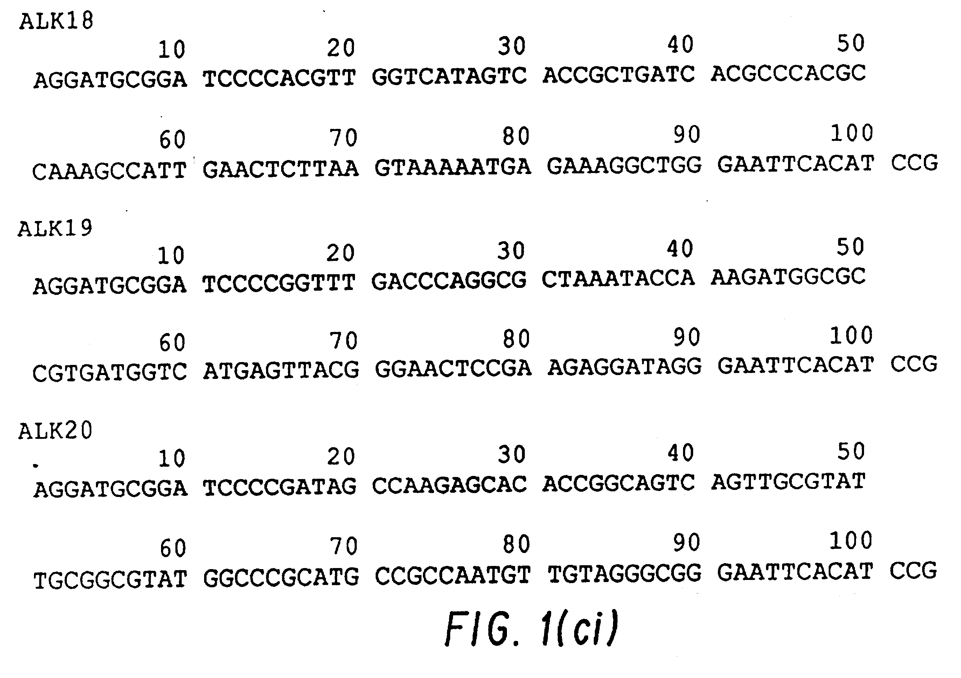

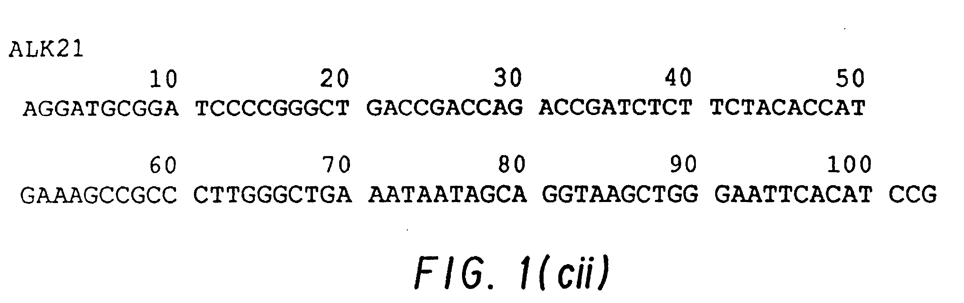

Twenty one synthetic oligonucleotides [Figure 1(a)-(c)] were designed and individually cloned into a custom designed plasmid vector (pWM500). Each synthetic oligonucleotide was then cut from the vector using *Fok*I restriction endonuclease. The resulting DNA fragments were designed such that, after cleavage from the vector, the protruding ends of each fragment had unique sequences which allowed the ligation of all 21 fragments in one reaction to produce a synthetic *phoA* gene of approximately 1600 base pairs.

The synthetic gene included the *phoA* ribosome binding site for the initiation of translation (the mechanism by which the base sequence of the gene is translated into the amino acid sequence of a polypeptide chain) as well as the transcription terminator site sequence (at which the synthesis of the mRNA chain complementary to the gene is terminated). The synthetic gene was cloned into a synthetic plasmid vector, pWM518, the construction of which is described below (Example 11). The vector was designed to have a limited number of restriction sites to facilitate such procedures as cloning, expression and mutagenesis. The alkaline phosphatase expression levels from the plasmid carrying the synthetic *phoA* gene (pMA100) approached ten milligrams of protein from one liter of cells grown to an optical density of 1.5 at 550 nanometers. An *E. coli* strain deficient in chromosomal alkaline phosphatase gene was used for transformation and subsequent fermentation to produce the enzymes disclosed herein. Such a strain includes the MZ13b (F⁻ *lacX*74, Δ(brnQ^{R}, *phoA*⁻, *phoB*⁻, *proC*⁻)₂₄ tsxR, trpₐₘ, str^{R}, F80D (proC⁺, proB⁺)_{xpw3}, F80) *E. coli* strain as described by Inouye, et al., J. Mol. Biol., Volume 110, pages 75-87 (1977), which is incorporated by reference herein.

A biological assay was used to screen the resultant mutant *E. coli* organisms to determine which clones produced an alkaline phosphatase having an enhanced specific activity. The clones were grown in media containing the substrate 5-bromo-4-chloro-3-indole phosphate (BCIP). Colonies producing an alkaline phosphatase having a high specific activity were detected by the appearance of a blue color around the colonies due to the enzyme's reaction with the substrate.

High expression levels of alkaline phosphatase by host cells were detrimental to the color screening of colonies on indicator plates (small increases in expression level did not manifest themselves as color changes to deeper blue). Therefore, to aid in distinguishing the mutant enzymes from native-like enzymes, the plasmids' alkaline phosphatase expression levels were reduced by replacing the native *lacZ* ribosome binding site with a sequence that is homologous to the native ribosome binding site but which is less efficient in the initiation of translation. The resultant plasmid was designated pMA101. The *phoA* gene included a group of five random nucleotides upstream of the initiator ATG codon. As a result, expression of the enzyme was substantially reduced, and only a mutant-enzyme with a high specific activity would exhibit the dark blue color with the BCIP substrate. A library of clones was generated containing about 1,000 clones expressing different levels of alkaline phosphatase activity. A clone (pMA101) expressing an alkaline phosphatase activity appropriate for color screening was selected for further mutagenesis studies.

### Site-Directed Mutagenesis

An oligonucleotide-directed double-strand break repair method (i.e., bridge mutagenesis) was used to construct a synthetic plasmid vector as disclosed in Mandecki, et al., Proc Natl. Acad. Sci., Volume 83, pages 7177-7181 (1986). The use of this particular method, however, is not critical to the present invention. The method involves the introduction of mutations by cotransforming competent *E*. *coli* cells with a denatured linear plasmid and a synthetic oligonucleotide sequence which encodes a suitable mutation and which has two "arms" that are homologous to the plasmid DNA sequences in the vicinity of the plasmid break. Because only a single-stranded oligonucleotide sequence is used for mutagenesis, the method is especially advantageous for the introduction of degenerate sequences into plasmid DNA.

In the procedure, a synthetic oligonucleotide subsequence, that encoded a mutation in a subsequence of the alkaline phosphatase gene, was cloned into the *phoA* gene of the plasmid vector to modify rather than replace the *phoA* gene. The synthetic oligonucleotide subsequences were designed to carry to the target site either a defined or random codon sequence which typically corresponded to a twenty amino acid chain. To introduce a subsequence, the *E. coli* plasmid containing the *phoA* gene was either cleaved next to the site targeted for mutagenesis (i.e., cut with a restriction endonuclease that cleaved the plasmid only once, there being 25 such sites within the *phoA* gene), or the plasmid was cleaved with two enzymes to liberate a restriction fragment which overlapped the site to be mutated. The host cells were transformed by combining the cells with the cleaved plasmid, the synthetic oligonucleotide and DNA ligase. From a single transformation, 200 colonies were obtained.

Based upon color screening, which was followed by DNA sequencing and an analysis of purified protein, ten mutant *E*. *coli* strains were obtained which carried genes that expressed an alkaline phosphatase having an increased specific activity. The mutants were: Thr¹⁰⁰ > Val (i.e., Threonine at the 100 position was replaced with Valine); Thr¹⁰⁰ > lie; Lys³²⁸ > Arg; Val⁹⁹ > Ala; Asp¹⁰¹ > Ser; Ala¹⁰³ > Asp; Ala¹⁰³ > Cys; Thr¹⁰⁷ > Val; a double mutant Lys³²⁸ > Arg and Val⁹⁹ > Ala; and a fortuitous double mutant Val³⁷⁷ > Ala and Ser⁴¹⁵ > Gly.

Alternative methods of preparing plasmid cloning vehicles which include such genetic mutations can also be used, such as those described by Polisky et al., Proc, Natl. Acad. Sci. USA, Volume 73(11), pages 3900-3904 (1976); U.S. Patent Number 4,375,514; and U.S. Patent Number 4,704,362.

### Analysis Of Alkaline Phosphatase Mutants

The analyses for specific activity and temperature stability were performed on highly purified protein material. In brief, the purification process involved the release of the periplasmic protein by the formation of spheroplasts, ammonium sulphate precipitation and chromatofocusing chromatography. The measurements of specific activity and the Michaelis constant relied upon monitoring the kinetics of the conversion of an enzyme substrate (e.g., p-nitrophenyl phosphate) to form a chromogenic product. Temperature stability was measured by monitoring the decay of enzyme activity at different temperatures.

The resultant enzymes and their properties are presented in Table 1. All of the mutant *E. coli* strains expressed alkaline phosphatase enzymes having specific activities that were higher than that of the native enzyme (as represented by pMA100). Although each of the mutant enzymes were less temperature stable than the native enzyme, they were all significantly more temperature stable than the mammalian enzyme, calf-intestinal alkaline phosphatase.

**Table 1**

| Constructed Mutants * | | | | |
|---|---|---|---|---|
| Plasmid | Mutation | Specific activity (µMole/mg/min) | Kₘ (µM) | Temperature stability (half-life) |
| pMA100 | wild-type | 60 | 30 | 6 min. at 95°C |
| pMA110 | Val³⁷⁷ > Ala | 90 | 23 | 7 min. at 80°C |
| | Ser⁴¹⁵ > Gly | | | |
| pMA111 | Thr¹⁰⁰ > Val | 123 | 20 | 21 min. at 85°C |
| pMA112 | Thr¹⁰⁰ > Ile | 123 | 20 | 10 min. at 85°C |
| pMA113 | Lys³²⁸>Arg | 220 | 94 | 10 min. at 85°C |
| pMA114 | Val⁹⁹ > Ala | 205 | 22 | 15 min. at 80°C |
| | | | | 3 min. at 85°C |
| pMA115 | Asp¹⁰¹ > Ser | 290 | 56 | 14 min. at 80°C |
| | | | | 2 min. at 85°C |
| pMA116 | Lys³²⁸ > Arg | 190 | 74 | 5 min. at 75°C |
| | Val⁹⁹ > Ala | | | <1 min. at 85°C |
| pMA117 | Ala¹⁰³>Asp | 133 | 144 | 22 min. at 85°C |
| pMA118 | Ala¹⁰³> Cys | 105 | 75 | 29 min. at 85°C |
| pMA119 | Thr¹⁰⁷ > Val | 240 | 102 | 7 min. at 85°C |
| calf-intestinal | alkaline phosphatase | 1800 | 10 | 9 min. at 65°C |

| | | | | |
|---|---|---|---|---|
| * disclosed in EP-A-0 441 252 | | | | |

### Binding Assays Using Alkaline Phosphatase Labels

Before proceeding further with the description of the use of alkaline phosphatase as a label in binding assays, a number of terms will be defined. A variety of assay techniques in which the novel labels are advantageously used are also described.

"Specific binding member" refers to a member of a specific binding pair, i.e., two different molecules wherein one of the molecules through chemical or physical means specifically binds to the second molecule. In addition to antigen and antibody specific binding pairs, other specific binding pairs include, as examples without limitation, biotin and avidin, carbohydrates and lectins, complementary nucleotide sequences (as in DNA hybridization reactions), complementary peptide sequences, effector and receptor molecules, enzyme cofactors and enzymes, enzyme inhibitors and enzymes, a peptide sequence and an antibody specific for the sequence or the entire protein, polymeric acids and bases, dyes and protein binders, peptides and specific protein binders (e.g., ribonuclease, S-peptide and ribonuclease S-protein), and the like.
Furthermore, specific binding pairs can include members that are analogs of the original specific binding member, for example an analyte-analog. If the specific binding member is an immunoreactant it can be, for example, an antibody, antigen, hapten, or complex thereof, and if an antibody is used, it can be a monoclonal or polyclonal antibody, a recombinant protein, a mixture of two or more antibodies, an antibody fragment, or mixtures thereof, as well as, a mixture of an antibody and other specific binding members. The details of the preparation of such antibodies and their suitability for use as specific binding members are well known to those skilled-in-the-art.

"Analyte" refers to the compound or composition to be detected or measured in the assay. The analyte can be any substance for which there exists a naturally occurring analyte-specific binding member or for which an analyte-specific binding member can be prepared. Analytes include, but are not limited to toxins, organic compounds, proteins, peptides, amino acids, nucleic acids, hormones, steroids, vitamins, drugs (including those administered for therapeutic purposes as well as those administered for illicit purposes), and metabolites of or antibodies to any of the above substances. The term "analyte" also includes any antigenic substances, haptens, antibodies, and combinations thereof which are of interest in immunoassays. The reagents and methods of the present invention can also be designed to determine food product and environmental analytes of interest.

"Indicator reagent" refers to a specific binding member attached to a label. The indicator reagent produces a detectable signal that is related to the amount of analyte in the test sample. In general, the indicator reagent is detected or measured after it is immobilized upon a solid phase material, but free or unbound indicator reagent can also be detected or measured to determine the result of an assay. The specific binding member of the indicator reagent can be a member of any specific binding pair as described above. In the present invention, the label component of the indicator reagent is a synthetic alkaline phosphatase having an enhanced specific activity. The enzyme label is used to convert an enzyme substrate into a detectable product. The product can be detected by visual or instrumental means. Amplification of the detectable signal can also be obtained by reacting the enzyme with one or more substrates or additional enzymes to produce a detectable reaction product.

"Capture binding member" refers to a specific binding member which can directly or indirectly bind the analyte or indicator reagent and which is typically bound or is capable of being bound to a solid phase, by covalent, noncovalent, adsorption or non-specific mechanisms, or is capable of being precipitated, such that the capture binding member can be separated from the test sample and other assay reagents.

"Capture reagent" refers to a capture binding member which is directly or indirectly attached to a solid phase material to enable the separation of the capture binding member, and analyte or indicator reagent that is bound thereto, from unbound analyte and assay reagents. Typically, the attachment of the capture binding member to the solid phase is substantially irreversible and can include covalent mechanisms. The capture reagent of the present invention, however, is not limited to a capture binding member bound to an insoluble solid phase material. In an agglutination assay, the capture reagent can include a capture binding member bound to a soluble carrier material such as bovine serum albumin and the like.

"Solid phase material" refers to any suitable chromatographic, bibulous, porous or capillary material or other conventional solid material, well known to those skilled-in-the-art, used to immobilize specific binding members. In the present invention, the solid phase material can include a fiberglass, cellulose or nylon pad for use in a flow-through assay device having one or more layers containing one or more of the assay reagents; a dipstick for a dip and read assay; a test strip for chromatographic (e.g., paper or glass fiber) or thin layer chromatographic (e.g., nitrocellulose) techniques in which one or all of the reagents are contained in separate zones of a single strip of solid phase material; or an absorbent material well known to those skilled in the art. The solid phase material can also include, without limitation, polyacrylamide beads, polystyrene beads or tubes, magnetic beads, a microtitre plate or a glass or plastic test tube.

Natural, synthetic or naturally occurring materials that are synthetically modified, can be used as a solid phase material including polysaccharides, e.g., cellulose materials such as paper and cellulose derivatives such as diazobenzyloxymethylcellulose, nitrocellulose, 2-aminophenylthioethercellulose, and cellulose acetate; silica; silicon particles; inorganic materials such as deactivated alumina, or other inorganic finely divided material uniformly dispersed in a porous polymer matrix, with polymers such as vinyl chloride, vinyl chloride polymer with propylene, and vinyl chloride polymer with vinyl acetate; cloth, both naturally occurring (e.g., cotton) and synthetic (e.g., nylon); porous gels such as silica gel, agarose, dextran, and gelatin; polymeric films such as polyacrylates; protein binding membranes; and the like. The solid phase material should have reasonable strength or strength can be provided by means of a support, and it should not interfere with the production of a detectable signal.

Optionally, the capture binding member of the capture reagent can be affixed to particles, e.g., microparticles. These microparticles can serve as the solid phase material and be retained in a column, suspended in a mixture of soluble reagents and test sample, or retained and immobilized by another solid phase base material. By "retained and immobilized" is meant that the microparticles, associated with the solid phase base material, are not capable of substantial movement to positions elsewhere within that material. The microparticles can be selected by one skilled in the art from any suitable type of particulate material including those composed of polystyrene, polymethylacrylate, polypropylene, polytetrafluoroethylene, polyacrylonitrile, polycarbonate or similar materials. The size of the microparticles is not critical, although it is preferred that the average diameter be smaller than the average pore size of the solid phase base material if such is used.

The present invention can also include a capture reagent that involves a capture binding member that is not initially attached to a solid phase material. Once complex formation occurs between the assay components, the solid phase can be used as a separation mechanism. For example, the reaction mixture can be contacted with the solid phase material, and the newly formed reaction complex(es) are retained by the solid phase material. Alternative methods can be used to perform this separation step, such as using a solid phase which itself binds to the capture binding member; affixing to the solid phase a binding member that is specific for the capture binding member; or affixing to the solid phase an agent, such as a charged substance, which will attract and bind an oppositely charged substance that has been bound to the capture binding member.

"Ancillary specific binding member" refers to a specific binding member used in addition to the specific binding members of the capture binding member and the indicator reagent which becomes a part of the detectable binding complex. One or more ancillary specific binding members can be used in an assay. For example, an ancillary specific binding member can be used in an assay where the indicator reagent is capable of binding the ancillary specific binding member which is in turn capable of binding the analyte.

"Test sample" typically refers to a naturally occurring or artificially formed liquid test medium suspected of containing the analyte of interest. The test sample is generally a biological fluid or a dilution thereof. Biological fluids from which an analyte can be determined include serum, whole blood, plasma, urine, saliva, amniotic and cerebrospinal fluids, and the like. The test sample can also include a solid material (e.g., hair, tissue, etc.) which has been modified to form a liquid test medium.

It will be appreciated by those skilled-in-the-art that the selection of any given binding member, ancillary binding member or solid phase material is generally not critical to the present invention. The materials are chosen to optimize the assay results for a given analyte or test sample.

The novel enzymes of the present invention are advantageously used in homogeneous binding assays, including competitive binding assay formats, and in heterogenous binding assays, including both sandwich and competitive binding assay formats. Heterogeneous binding assay techniques involve the use of a solid phase material to which a member of the binding reaction becomes bound. Prior to detecting the label which indicates the presence or amount of analyte in the test sample, the immobilized reaction component is separated from excess sample and assay reagents by removing the solid phase from the reaction mixture.

In a solid phase sandwich assay, the capture reagent typically involves a capture binding member which has been bound to a solid phase material. For example, the specific binding member can be an immobilized antibody which will bind to an antigen-analyte in the test sample, or the specific binding member can be an immobilized antigen which will bind to an antibody-analyte in the test sample. The capture reagent is contacted to a test sample, suspected of containing the analyte, and to an indicator reagent comprising a second specific binding member that has been labeled; for example, a labeled anti-analyte antibody. The reagents can be mixed simultaneously or added sequentially, either singly or in combination. A binding reaction results in the formation of a capture reagent/analyte/indicator reagent complex. The assay can also comprise the step of separating the resultant complex from the excess reagents and test sample. The complex retained on the solid phase material is detected by examining the solid phase for the indicator reagent. If analyte is present in the sample, then label will be present on the solid phase material. The amount of label which becomes associated with the solid phase is directly proportional to the amount of analyte in the sample.

The assays of the present invention can be carried out using any of the sandwich assay formats, including the forward, reverse and simultaneous techniques. Typically, a forward assay involves the contact of the test sample to the capture reagent followed by an incubation period which is in turn followed by the addition of the indicator reagent. A reverse assay involves the addition of the indicator reagent to the test sample followed by the addition of the capture reagent after an incubation period. A simultaneous assay involves a single incubation step as the capture reagent and indicator reagent are both contacted to the test sample at the same time.

In addition, the novel enzymes of the present invention can be used in an indirect sandwich assay with the formation of a complex of capture reagent/analyte/analyte-specific binding member/indicator reagent. In this case, the additional analyte-specific binding member is the ancillary specific binding member.

Competitive assays can also be carried out using the novel enzymes of the present invention. In a solid phase competitive assay, the capture reagent again typically involves a capture binding member which has been affixed to a solid phase material and which is contacted with both test sample and an indicator reagent. The indicator reagent, however, can be formed from an analyte or analyte-analog which has been conjugated with a label. A binding reaction occurs and results in the formation of complexes of (1) immobilized capture reagent/analyte complex and (2) immobilized capture reagent/indicator reagent complex. Alternatively, the immobilized specific binding member can be an analyte or analyte-analog with which the test sample analyte competes for binding to the indicator reagent. In the competitive assay, the amount of label which becomes associated with the solid phase is inversely related to the amount of analyte in the sample. Thus, a positive test sample will generate a decrease in signal.

In these binding assays, the presence or amount of the analyte in the test sample is usually determined by detecting the presence or amount of the label which has become associated with the solid phase, although free or unbound indicator reagent may also be-detected. In the competitive assay, the more analyte present in the test sample the lower the amount of label present on the solid phase. In the sandwich assay, the more analyte present in the sample the greater the amount of label present on the solid phase.

As specifically described in the following examples, several of the recombinant enzymes were chemically coupled to different immunoglobulins to form conjugates for use in enzyme immunoassays (EIA's). For example, mutant enzymes expressed by plasmids pMA110, pMA111, pMA112, pMA113 and pMA115 were conjugated via various heterobifunctional coupling reagents to an anti-alpha-fetoprotein monoclonal antibody (anti-AFP antibody). These antibody/enzyme conjugates were subsequently used in an EIA as indicator reagents. For example, an AFP standard curve was prepared in an assay using an anti-AFP antibody conjugated with the alkaline phosphatase enzyme from the mutant plasmid pMA113. The pMA113 enzyme/antibody indicator reagent produced a standard curve which was comparable to that generated with a mammalian enzyme/antibody indicator reagent under similar assay conditions. The use of heterobifunctional coupling reagents is well-known in the art, and no specific heterobifunctional coupling reagent is essential to the novel indicator reagents of the present invention as defined in Claim 6.

The alkaline phosphatase enzymes disclosed herein have also been chemically coupled to several other proteins such as cancer antigen-125 and anti-carcinoembryonic antigen antibodies, a cancer antigen 19-9 Fab fragment and a human chorionic gonadotropin antibody. All have been used successfully in automated EIA's wherein the presence of alkaline phosphatase is detected upon the addition of an enzyme substrate to produce a detectable product and thereby indicate the presence or amount of a reaction component. Many alkaline phosphatase substrates are available for use in the binding assays which employ the alkaline phosphatase labels. Commonly used substrates are p-nitrophenyl phosphate (pNPP), 5-chloro-4-bromo-3-indolyl phosphate (XP), and methylumbelliferyl phosphate (MUP). The MUP and pNPP substrates are frequently used in immunoassays. As an alternative embodiment of the binding assay, the indicator reagent can comprise a specific binding member labeled with an enzyme substrate and the alkaline phosphatase enzyme is added to produce the detectable signal.

The construction and properties of the mutant of the invention is disclosed in Example 12. Examples 1-11 are references examples disclosed in EP-A-0 441 252.

### Example 1

### Preparation Of Mutant And Synthetic Wild-Type phoA Genes

### a. Oligonucleotide Synthesis

Although it was not essential to the production of the enzymes disclosed herein, a synthetic *phoA* gene was constructed to facilitate the mutagenesis of the alkaline phosphatase gene and the expression of that gene. The structure of the synthetic gene was based upon the sequence for the wild-type *E. coli* alkaline phosphatase gene disclosed by Chang et al., Gene 44, 121-125 (1986).

The gene was engineered with an *E. coli* codon preference and unique restriction sites at intervals of approximately 50-100 base pairs. To construct the *phoA* gene, the *Fok*I method of gene synthesis was used, as disclosed by Mandecki et al., Gene, 68, 101-107 (1988).

The *phoA* gene sequence was divided into twenty-one oligonucleotide subsequences, each 73 bases in length. An additional thirty bases corresponding to *Fok*I arms were added to each subsequence, to overlap and anneal to the cut plasmid DNA on each side of the cleavage site.

The oligonucleotides were synthesized on an Applied Biosystem 380B synthesizer (Applied Biosystems, Foster City, CA) using 5'-dimethoxytrityl nucleoside β-cyanoethyl phosphoramidites. The oligonucleobdes were purified using gel electrophoresis (also described in Gene, 68, 101-107, 1988) on 10% polyacrylamide gels (10% polyacrylamide, 7.0 M urea, and 1 x TBE [89 mM Tris-borate, 89 mM boric acid, 2.0 mM ethylenediamine tetraacetic acid (EDTA)]). The DNA was visualized by UV shadowing, and the band corresponding to a 103 base pair subsequence was excised from the gel. The oligonucleotide was eluted from the portion of excised gel with one milliliter of Maxam's Elution Buffer (0.5 M ammonium acetate and 1 mM EDTA) at 37°C for 16 hours. To remove residual polyacrylamide, the eluted oligonucleotide was passed through a filter (0.2 µM Centrex filter; Schleicher & Schuell, Inc., Keens, NH). The purified oligonucleotide was precipitated with five volumes of ethanol, resuspended in water (50 µL) and quantitated using a Beckman DU-7 spectrophotometer (Beckman Instruments, Palo Alto, CA). The sequences of the synthetic oligonucleotides are presented in Figure 1(a)-(c).

### b. Cloning Of DNA

The cloning of the synthetic oligonucleotides into a plasmid vector was accomplished by the bridge mutagenesis protocol as described above. The plasmids chosen for cloning were pWM500 and pWM501, as described above.

The cloning vectors were cleaved with *Sma*I restriction endonuclease. A cleaved plasmid (approximately 50 ng) was mixed with an oligonucleotide subsequence (20 pmoles) in 30 microliters of denaturation buffer (10 mM KCl, 5 mM Tris-HCl pH 8.0, 5 mM MgSO₄, and 0.5 mM dithiothreitol). The samples were heated at 100°C for three minutes in a boiling water bath and were then cooled to room temperature for five minutes. The samples were then mixed with chilled competent JM83 host cells (100 µl: *ara*, *Δ*[*lac-proAB*]. *strA, thi*, F80d, *lacZΔM15*) as described by Vieira et al., Gene, Volume 19, pages 259-268 (1982). The JM83 cells were prepared by the CaCl₂ procedure as disclosed by Mandel, et al., in J. Mol. Biol., Volume 53, pages 159-162 (1970).

The mixture was chilled on ice for five minutes, followed by a three minute heat-shock at 37°C. Approximately two milliliters of luria broth (LB) media (containing per liter: bacto-tryptone 10 g; bacto-yeast extract 5 g; and NaCI 10 g; at pH 7.5) was added to the transformation mixture, and the mixture was incubated at 37°C for one hour. The transformed cells were then concentrated by centrifugation in a Sorvall GLC-2B table-top centrifuge (at 4,000 rpm for five minutes). The cells were resuspended in LB media (100 µl) and plated on LB plates containing 5-bromo-4-chloro-3-indoyl-D-galactoside (1.6 mg) and ampicillin (LB media/ampicillin; 100 µl at 25 mg/mL) for selection of colonies with ampicillin resistance, i.e., bacterial cells containing plasmid. The plates were incubated at 37°C for 15 hours, and transformants were scored by β-galactosidase color assay.

Four individual cell colonies were picked for each of the 21 cloned oligonucleotides. Single colonies were inoculated into LB media (0.5 ml) containing ampicillin (100 µg/ml). The cultures were grown at 37°C with constant agitation for five hours. Twenty-one individual cell cultures (i.e., one culture corresponding to each oligonucleotide subsequence) were then pooled and added to one liter of LB media containing ampicillin and were grown for 2.5 hours to an optical density of 0.65 at 600 nanometers. Four separate cultures of the pooled subsequences were made in an attempt to avoid subclones containing additional mutations which may have arisen during the synthesis of the oligonucleotide subsequences. The cultures were amplified with chloramphenicol (final concentration 25 mM) and incubated at 37°C for 16 hours as disclosed by Frenkel, et al., DNA, Volume 5, pages 539-544 (1986),

### c. Construction Of DNA Fragment Inserts

The transformed cells were harvested by centrifugation (10,000 x g for five minutes at 4°C.) The cells were lysed, and the plasmid DNA was purified on a cesium chloride gradient as disclosed by Birnboim, et al., Nucleic Acids Research, Volume 7, page 1513 (1979).

The purified plasmid DNA of the four pooled preparations was digested with *Fok*I to obtain DNA fragment inserts. Approximately 250 micrograms of pooled subsequence plasmid DNA were digested with 200 units of *Fok*I in 500 microliters of a buffer solution (20 mM KCl, 10 mM Tris-HCl pH 7.5, and 10 mM MgCl₂) for 2.5 hours at 37°C. The digests were electrophoresed on a 6% polyacrylamide gel, and the fragment corresponding to a 73 base pair oligonucleotide subsequence was excised. The fragment elution was performed substantially in accordance with the procedure used for the oligonucleotide purification as described in Example 1. a., above.

The purified *Fok*I fragments were resuspended in water (15 µl). An aliquot (0.5 µl) of each of the four samples was electrophoresed on a 0.8% agarose gel to approximately determine the DNA concentrations that were produced. Based on estimated quantitations, 4.5 micrograms of pooled *Fok*I fragments were obtained from each sample.

### d. Mutant E. coli Strains

To produce a highly expressed, functional alkaline phosphatase protein, the twenty-one fragment inserts were ligated with the synthetic plasmid, pWM518. In this expression system, the synthetic *phoA* gene was under the control of the lactose promoter and the ribosome binding site of the native *phoA* gene. Fifty nanograms of each fragment were used for ligation with 150 nanograms of *Bam*HI/*Hind*III cleaved vector.

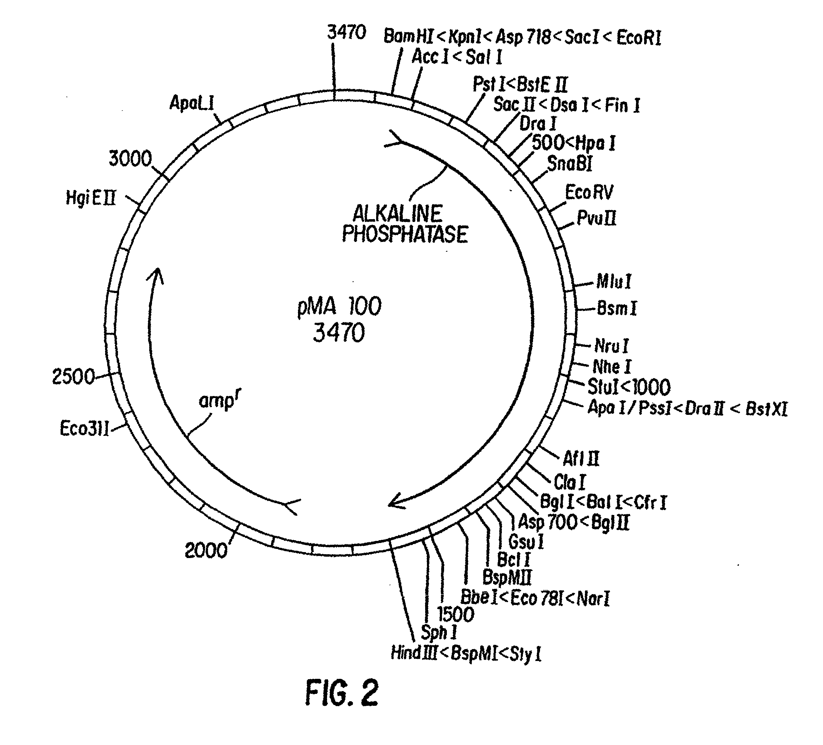

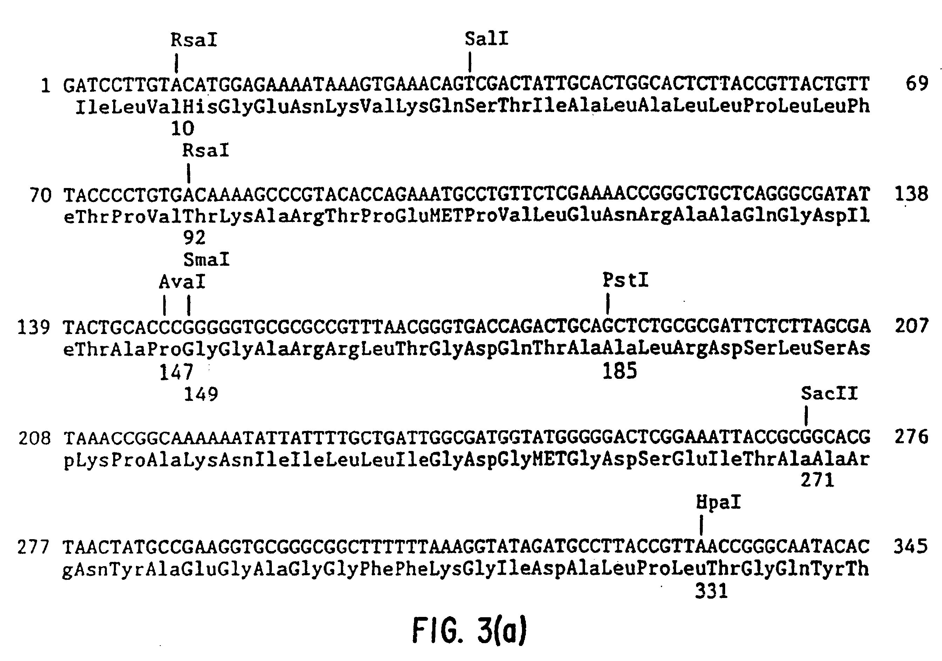

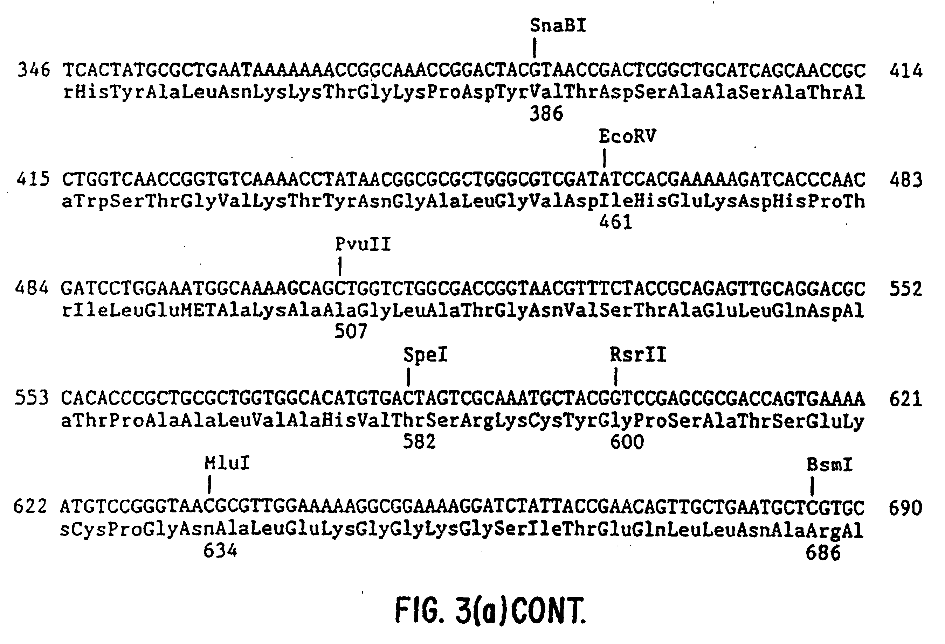

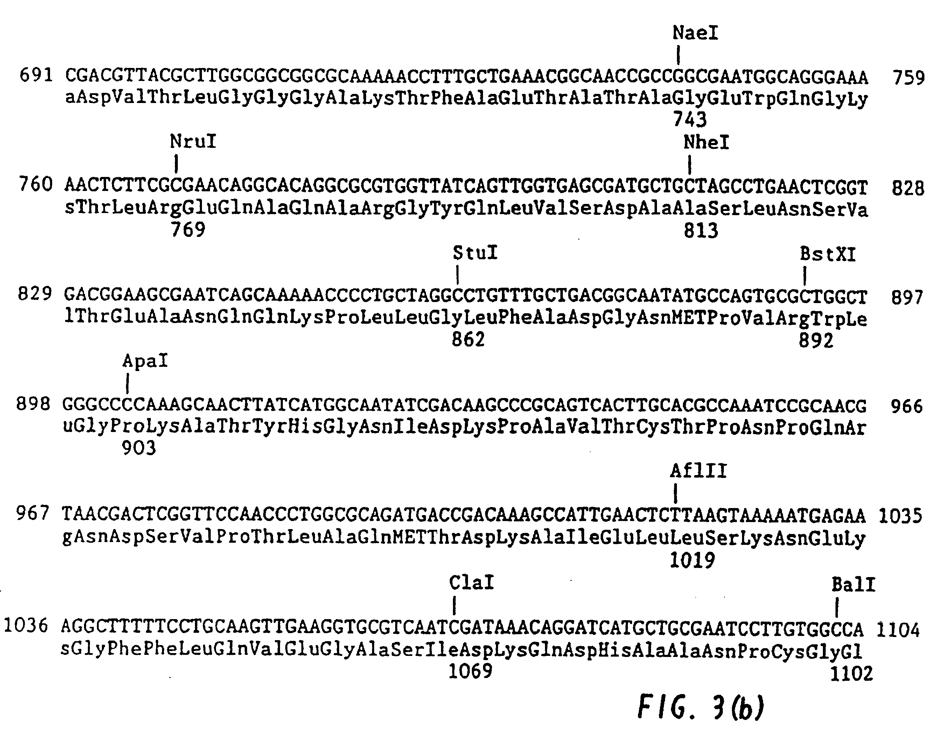

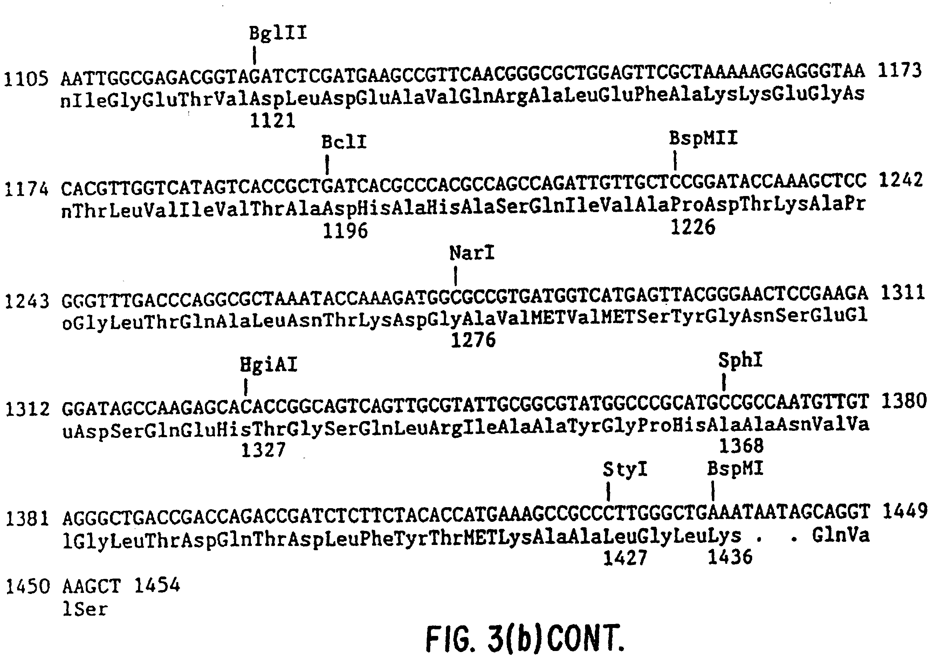

To be assured of obtaining a *phoA* gene without mutations, which was designated pMA100, equal aliquots of the four plasmid preparations were combined. The restriction map of pMA100 is illustrated in Figure 2, and the nucleotide and amino acid sequences of the synthetic, wild-type *E. coli* alkaline phosphatase, having unique restriction sites, are illustrated in Figure 3(a)-(b). The ligation of the oligonucleotide subsequences and vector was performed in a ligation mixture (10 µl: 60 mM Tris [pH 7.5]; 5 mM MgCl₂; 0.4 mM adenosine triphosphate; and 10 mM dithiothreitol). Prior to the addition of the enzyme ligase (T4 DNA ligase), the samples were incubated at 42°C for 15 minutes and then placed at 4°C for 1.5 hours. After the addition of ligase, the samples were incubated at 0°C for 16 hours. The ligation reaction was tested for completion by analysis on a 5% acrylamide gel (1/50 bis-acrylamide). The migration of the ligated material revealed a 3.5 kilobase fragment corresponding to the full length *phoA* gene in pWM518, as well as a ladder of partialy ligated and unligated fragments. The ligated mixture was then transformed into SCS-1 host cells (F⁻, *recA1*, *gyrA96*, *thi*, *hsdR17*, (rₖ⁻mₖ⁺), *supE44, relA1*, l⁻: Stratagene, San Diego, CA). The transformation procedure was performed in accordance with the procedure described by Hanahan, J. Mol. Biol., Volume166, pages 557-580 (1983). SCS-1 cells (100 µl) were thawed and aliquoted into a prechilled polypropylene tube (15 mL; Falcon 2059, Fisher Scientific, Pittsburgh, PA). β-Mercaptoethanol (1.4 M, 1.7 µl) was added to the cells which were gently swirled for ten minutes at 0°C. One nanogram of plasmid DNA was added to the cells to achieve the highest transformation efficiency, i.e., the highest number of host cells containing replicated plasmid. Different dilutions of the mixture, as presented in Table 2, were studied. The samples were incubated on ice for thirty minutes, heat pulsed in a 45°C water bath for 45 seconds and placed on ice for two minutes. SOC medium (0.9 ml) was added, and the samples were incubated (37°C for one hour while shaking at 225 rpm). (SOC medium contained, per liter, bacto-tryptone 20 g, bacto-yeast extract 5 g, 10 mM NaCI, and 2.5 mM KCI, mixed with 2 M filter-sterilized Mg solution [1 ml/100 ml of SOC; 1 M MgCl₂ with 1 M MgSO₄], and 2 M filter-sterilized glucose solution [1 ml/100 ml of SOC].) After incubation, the cells were concentrated by centrifugation at 1000 rpm for 10 minutes. The resultant pellet was resuspended in SOC (200 µl) and plated on LB media containing ampicillin (50 µg/ml).

Colonies which synthesized alkaline phosphatase were identified by their blue color through the use of BCIP, suspended in water [100 µl; 20 mg/ml]), which was added to each plate. The transformation efficiencies are listed in Table 2. SCS-1 cells were also transformed with pUC9 plasmid DNA, a conventionally used control, (Bethesda Research Laboratories, Gaithersberg, MD) for a comparative qualitative determination of the transformation efficiencies. The overall transformation efficiency for the SCS-1 host cells was approximately 2 x 10⁷ colonies/µg pUC9 DNA. The efficiency as described by Stratagene is greater than 1 x 10⁹ colonies/µg DNA.

**Table 2**

| Transformation Efficiencies | | | |
|---|---|---|---|
| Samples | Ligation mixture (1.0 µl) | DNA Concentration | Number of Colonies |
| Sample 1 | undiluted | 120.0 ng | 3 blue, 362 white |
| Sample 2 | 1:10 dilution | 12.0 ng | 3 blue, 124 white |
| Sample 3 | 1:25 dilution | 4.8 ng | 0 blue, 53 white |
| Sample 4 | 1:50 dilution | 2.4 ng | 0 blue, 16 white |
| cut vector ligated | undiluted | 15.0 ng | 68 white |
| pUC9 | | 1.0 ng | ∼20,000 white |

Because only six transformants (six blue colonies) demonstrated the presence of a functional synthetic *phoA* gene, the transformation protocol was repeated to obtain more clones to analyze for the native sequence. A total of 16 colonies were picked from the transformed SCS-1 cells, and plasmid DNA was isolated by miniprep procedure as disclosed by Birnboim et al., Nucleic Acids Res. 7, 1513 (1979). An aliquot of each sample was digested with *Eco*RI and *Hind*III and was electrophoresed on a 0.8% agarose gel along with molecular, weight markers (DNA/*Hind*III fragments, FX174 RF DNA/*Hae*III fragments and one kilobase DNA ladder; Bethesda Research Laboratories, Gaithersburg, MD). Of the 16 samples digested, all appeared to contain a fragment insert of 1.4 kilobases which corresponds in size with the full length alkaline phosphatase gene.

Four clones were picked from the 16 samples, and plasmid DNA was individually isolated and purified on a CsCl gradient as disclosed by Radloff, et al., Proc. Natl. Acad. Sci., Volume 57, pages1514-1521 (1967).

The samples were sequenced by the Sanger dideoxy method using multiple overlapping sequencing primers, as disclosed by Sanger, et al., Proc. Natl. Acad. Sci., Volume 74, pages 5463-5467 (1977).

All four samples contained the same mutations; C to T at 1191, T to C at 1220 and A to G at 1333. The first mutation was silent, but the remaining changes resulted in the mutations of Val³⁷⁷ > Ala and Ser⁴¹⁵ > Gly. The resultant clone was designated pMA110.

### e. Preparation Of A Synthetic Wild-Type E. coli Strain

A wild-type *phoA* gene (pMA100) was used as a basis for subsequent DNA modifications which encoded an alkaline phosphatase having an enhanced specific activity. In addition, alkaline phosphatase from a synthetic wild-type organism was useful in the evaluation of the mutant enzymes as well as their comparison to commercially available *E. coli* alkaline phosphatase. To produce the synthetic wild-type *phoA* gene, the existing genetic mutations in the pMA110 plasmid needed to be repaired. Synthetic oligonucleotides, made substantially in accordance with the protocol described in Example 1. a., were used to replace the mutated sequences with wild-type sequences. Due to the positioning of the mutations, a *Bgl*II/*Sph*I fragment corresponding to 246 base pairs needed to be replaced.

The pMA110 plasmid (approximately 10 µg) was digested with *Bgl*II (75 units) in 1x medium salt buffer (100 µl; 100 mM NaCl, 50 mM Tris-HCl pH 7.5 and 10 mM MgCl₂) for 16 hours at 37°C. To test for complete digestion, an aliquot was electrophoresed on a 0.8% agarose gel. The salt concentration was increased to 150 mM NaCI, and the DNA was further digested with *Sph*I (60 units) at 37°C for 16 hours. The resultant 3.2 kilobase plasmid fragment was purified on a 5% polyacrylamide gel (1/50 bis-acrylamide), and the DNA was extracted substantially in accordance with the procedure described above in 1. a.

The excised mutant *Bgl*II/*Sph*I fragment was replaced with three complementary synthetic oligonucleotides corresponding to the native sequence of the *Bgl*II/*Sph*l fragment (each approximately 80 bases in length), comprising 247 base pairs. Approximately four pmoles of each synthetic oligonucleotide were kinased using T4 DNA kinase (3.0 units; Bethesda Research Laboratories, Gaithersburg, MD) at 37°C for 30 minutes in 1x ligation buffer (15 µl; 60 mM Tris-HCl pH 7.5, 5 mM MgCl₂, 0.4 mM ATP), as disclosed by Richardson et al., Proc. Natl. Acad. Sci., 2, 815 (1971).

The complementary oligonucleotides were annealed through incubation at 70°C for five minutes and slow cooling to 4°C for 1.5 hours. The annealed oligonucleotide was then ligated to the 3.2 kilobase purified synthetic plasmid. The ligation, transformation and incubation procedures were performed substantially in accordance with the procedures described above, with the exception that the resultant synthetic plasmids were cloned into MZ13b host cells.

Approximately 200 blue colonies and 120 white colonies were obtained. Four blue colonies were selected and a miniprep DNA was prepared substantially in accordance with the procedures described above. The region corresponding to the replaced or repaired oligonucleotide subsequence was sequenced by the Sanger dideoxy method substantially in accordance with the procedures described above. All of the sequenced clones contained the wild-type *phoA* sequence. In comparing the color intensities of colonies obtained from native and mutant alkaline phosphatase, the mutant colonies were darker blue than the native colonies.

### Example 2

### Modified Expression Of Native phoA For Screening Of Mutants

High sensitivity color screening of those alkaline phosphatase mutants having an increased specific activity was difficult due to the intense color exhibited by native alkaline phosphatase. To distinguish mutants from native alkaline phosphatase, it was necessary to decrease the expression of native *phoA* (i.e., it was necessary to obtain a light blue native colony). Expression levels of alkaline phosphatase were reduced by replacing the Shine-Delgarno Sequence (GGAGA) of *phoA* with a degenerate sequence and screening for faint blue colonies by use of BCIP substrate.

To remove the native ribosome binding site, pMA100 (15 µg, from Example 1. e.) was digested in 1x medium salt buffer with 75 units of *Bam*HI at 37°C for 16 hours. The salt concentration was increased to 150 mM NaCl, and the DNA was further digested with 75 units of *Sal*l at 37°C for 16 hours. The ribosome binding site was replaced by bridge mutagenesis, as described in Example 1, by providing an oligonucleotide carrying a degenerative sequence at the appropriate position. Approximately 1,000 clones, expressing varying levels of alkaline phosphatase activity, were generated.

Three colonies, ranging from very light blue to medium blue, were isolated, and plasmid DNA was prepared substantially in accordance with the protocol described in Example 1. To determine the structure of the mutated ribosome binding site generated in each sample, Sanger dideoxy sequencing was again used substantially in accordance with the protocol described in Example 1. The sequencing results demonstrated that the transformants which were sequenced had either 1) a total deletion of the ribosome binding site or 2) a ribosome binding site containing the sequence ATGGC or CAATA. The sample corresponding to the ATGGC ribosome binding site exhibited the faintest blue color and was given the designation pMA101. The further mutagenesis of the *phoA* gene for increased specific activity was then performed and assayed using the pMA101 synthetic plasmid vector containing native *phoA* with a mutated ribosome binding site.

### Example 3

### Mutagenesis Of Native Alkaline Phosphatase

To increase the specific activity of alkaline phosphatase, the mutagenesis of the enzyme was directed toward the enzyme's active region or to an area within about 10 Å to about 20 Å from the catalytic residue Ser¹⁰². The amino acids targeted for mutagenesis were Val⁹⁹, Thr¹⁰⁰, Asp¹⁰¹, Ala¹⁰³, Thr¹⁰⁷ and Lys³²⁸. The amino acids Val⁹⁹, Thr¹⁰⁰, Asp¹⁰¹, Ala¹⁰³ and Thr¹⁰⁷ were of particular interest for mutagenesis due to their proximity to the catalytic residue Ser¹⁰². Also of interest was Lys³²⁸, which carries a positive charge and is in the immediate vicinity of the enzyme's active site.

Mutagenesis of the Val⁹⁹, Thr¹⁰⁰ and Asp¹⁰¹ amino acids was accomplished by bridge mutagenesis at the *Sna*BI restriction site of pMA101, and at the *Cla*I restriction site for Lys³²⁸. For Ala¹⁰³ and Thr¹⁰⁷, bridge mutagenesis was accomplished after digestion of the pMA101 plasmid with *Sna*BI and *Eco*RV. Synthetic oligonucleotides were made, as described in Example 1, wherein each synthetic oligonucleotide contained a degenerate sequence for the amino acid to be mutated.

The standard procedure for bridge mutagenesis underwent a 5-fold increase in scale to produce a library of clones containing all possible amino acid substitutions for each residue mutated. The pMA101 vector (250 ng, from Example 2) was completely digested with either *Sna*BI (for mutations at 99, 100 and 101), *Cla*I (for the mutation at 328) or *Sna*BI and *Eco*RV (for the mutation at 103 and 107). Digested vector and one of the synthetic oligonucleotides (100 pmoles) were mixed and heated for three minutes at 100°C. The sample was cooled for five minutes and was added to competent JM83 cells (500 µl). The transformation mixtures were incubated on ice for five minutes and then heat shocked at 37°C for three minutes. LB media (2.0 ml) was added to each sample, and the samples were incubated at 37°C for 60 minutes. Cells were pelleted by centrifugation, resuspended in LB media (100 µl), and spread on LB plates (containing 100 µg/ml ampicillin and 1.0 µg/ml BCIP). The plates were incubated for 16 hours at 37°C. Due to the decreased expression of *phoA* caused by the mutated ribosome binding site, the plates were incubated for an additional six hours at room temperature. Colonies with an intense, dark blue color, as compared with background, were scored as *phoA* mutants having an increased specific activity.

Blue colonies were selected and miniprep DNA prepared for Sanger dideoxy sequencing substantially in accordance with the procedure described in Example 1. The mutation results are presented in Table 3. To obtain accurate data on the specific activity of the mutants, as compared to native alkaline phosphatase, it was also necessary to obtain purified protein from each sample.

**Table 3**

| Mutagenesis Results | | |
|---|---|---|
| Amino Acid Mutated | Number of Colonies | Codons |
| pMA114 | 260 dark blue | GCA; GCT |
| Val⁹⁹ > Ala | 380 light blue | |
| | 3680 white | |
| | | |
| pMA111 and 112 | 23 dark blue | GTA/ ATC |
| Thr¹⁰⁰ > Val | 316 light blue | |
| Thr100 > Ile | 76 white | |
| | | |
| pMA115 | 130 dark blue | TCA; TCC |
| Asp¹⁰¹ > Ser | 260 light blue | |
| | 4200 white | |
| | | |
| pMA113 | 11 dark blue | CGA; AGA; |
| Lys³²⁸ > Arg | 320 light blue | CGT |
| | 16 white | |
| | | |
| pMA116 | 270 dark blue | GCT |
| Lys³²⁸ > Arg | 490 light blue | |
| Val⁹⁹ > Ala | 1340 white | |
| | | |
| pMA117 and 118 | 23 dark blue | GAT; TGT |
| Ala¹⁰³ > Asp | 141 light blue | |
| Ala¹⁰³ > Cys | 180 white | |
| | | |
| pMA119 | 15 dark blue | GTG |
| Thr¹⁰⁷ > Val | 87 light blue | |
| | 166 white | |

Table 3 presents the transformation efficiencies for those amino acid mutations which resulted in mutant alkaline phosphatase enzymes having enhanced specific activity. The dark blue colonies represented those alkaline phosphatase mutants having an enhanced specific activity, the light blue colonies were mutants with a specific activity comparable to native alkaline phosphatase, and the white colonies were mutants with a decreased specific activity. Because pMA113 (Lys³²⁸ > Arg) had a high specific activity, it was used for subsequent mutagenesis, for example pMA116 was produced by further changing Val⁹⁹ to Ala in the pMA113 plasmid. Codons obtained for each amino acid substitution were determined by Sanger didoxy sequencing.

### Example 4

### Purification Of Alkaline Phosphatase

To determine the enzyme kinetics of the native and the mutant alkaline phosphatase enzymes, sufficient quantities of the enzymes had to be obtained. For each sample, a two liter shaker flask fermentation procedure was performed. The starter culture was an overnight culture of a single colony in LB media/ampicillin (40 ml) containing 20 mM glucose. The addition of glucose repressed the organism's expression of alkaline phosphatase, which in the expression system of the present invention is controlled by the *lac* promoter (Magasanik B, The Lactose Operon, Cold Spring Harbor Laboratory, Cold Spring Harbor, NY, 189-219, 1970.) The culture was grown in a flask (250 ml) overnight at 37°C with vigorous shaking. The culture was centrifuged (4,000 x g) for ten minutes, the supernatant was discarded, and the pellet was resuspended in LB media/ampicillin (40 ml). A six liter flask, containing two liters of LB media/ampicillin, was inoculated with the overnight culture and was incubated for approximately six hours at 37°C with vigorous shaking.

The purification of alkaline phosphatase was conducted according to the following procedure. Upon reaching an optical density of 1.3 to 1.4 at 600 nanometers, the transformed cells were harvested by centrifugation (4800 rpm for 20 minutes at 4°C). To extract the alkaline phosphatase, the cells derived from two liters of culture broth were suspended in cold 0.15 M Tris-HCI buffer, pH 6.6 (80 ml; containing 0.9% NaCI and 6 mg/ml polymyxin B) as disclosed by Evans, et al., Journal of Infectious Diseases, Volume 133, pages S97-S102 (1976). After incubating for seven to ten minutes in a 37°C water bath, the cells were removed via centrifugation (8,000 x g for five minutes at 4°C).

The polymyxin B extract was brought to 85% saturation with the slow addition of solid ammonium sulfate at 4°C, and was then slowly stirred for 24 hours at 4°C. The resulting precipitate was harvested by centrifugation (8,000 x g for five minutes at 4°C) and was resuspended in 0.02 M Tris-HCl buffer (pH 8.0, containing 1 mM MgCl₂). Dialysis of the sample was carried out against 8 to 12 liters of 0.02 M Tris-HCl buffer (pH 8.0, containing 1 mM MgCl₂) at 4°C. Subsequently, the samples were concentrated 5- to 10-fold using an ultra-filtration cell (Amicon, Model 8050, Danvers, MA).

Chromatofocusing of the alkaline phosphatases was performed on a fast protein liquid chromatograph system (Pharmacia, Piscataway, NJ) using a Mono P HR 5/5 column and Polybuffer 74 and 96 (Sigma Chemical Company, St. Louis, MO). The column was equilibrated with 0.025 M bis-Tris-CH₃COOH (pH 6.9, containing 1 mM MgCl₂). The protein was eluted from the column with a Polybuffer-CH₃COOH solution (pH 5.5; containing 6% [v/v] Polybuffer 96, 6% [v/v] Polybuffer 74 and 1 mM MgCl₂). These conditions provided a linear pH gradient from 6.5 to 5.5 wherein the active protein eluted at a pH between 6.3 and 6.1. The Polybuffer was removed from the samples by gel filtration chromatography on Sephadex G-75 equilibrated in 20 mM Tris (pH 8.0, containing 1 mM MgCl₂). The resultant enzyme samples were then concentrated 3- to 6-fold using Amicon Centricon-30 microconcentrators.

### Example 5

### Analysis Of Purified Alkaline Phosphatase

Characterization of the purified proteins included sodium dodecyl sulfate polyacrylamide gel electrophoresis as disclosed by Laemmli, U.K., Nature, Volume 277, page 680 (1970).
Electrophoresis revealed a single major band which represented greater than 98% of the total stained protein and which corresponded to a molecular weight of 46,000 daltons, the expected size of a monomer of alkaline phosphatase. The electrophoretic mobility under these conditions for each of the genetically engineered alkaline phosphatases was identical to the commercially available *E. coli* alkaline phosphatase (Sigma).

The kinetic constants (Vₘₐₓ and Kₘ) of the resultant mutant enzymes were measured using the enzyme substrates pNPP (Sigma) and 4-methylumbelliferylphosphate (4-MUP; Boehringer Mannheim, Indianapolis, IN) in 1 M Tris-HCl buffer (pH 8.0, containing 1 mM MgCl₂) at 25°C. The conversion of the pNPP substrate to p-nitrophenol was monitored by following the change in absorption at 410 nm (e = 1.62 X 10⁴ M⁻¹ cm⁻¹) using a Hewlett-Packard 9153A UV spectrophotometer. The release of methylumbelliferone from the 4-MUP substrate was monitored fluorimetrically (I excitation = 340 nm; I emission = 465 nm; e = 5.9 x 10⁹ M⁻¹ cm⁻¹). Initial rates were determined graphically from the first 5-10% of the reaction (r > 0.997), Values of k_{cat} and Kₘ were obtained from the Lineweaver-Burk plots of six to eight points.

The results obtained for each of the enzymes are summarized in Table 4. All of the mutant enzymes possessed a higher specific activity than did the native alkaline phosphatase, using either pNPP or MUP as substrates. In the determinations made with pNPP, the increased specific activity of the enzyme ranged from a 1.6 to a 3.9 fold increase. The Km value is most dramatically increased for the enzyme produced by mutant plasmid pMA113, while remaining relatively unchanged or slightly improved for all others. Thus, the mutants possessed enhanced specific activity and acceptable, if not beneficial changes in Kₘ.

**Table 4**

| Kinetic Constants for the Mutant Enzymes | | | | |
|---|---|---|---|---|
| | pNPP Substrate | | 4-MUP Substrate | |
| | Vmax | Kₘ | Vmax | Kₘ |
| Alkaline Phosphatase | (µmol mg⁻¹ min⁻¹) | (µM) | (µmol mg⁻¹ min⁻¹) | (µM) |
| pMA100 | 56 | 30.1 | 106 | 9.3 |
| wild-type | | | | |
| | | | | |
| pMA110 | 90 | 23 | 182 | 14.4 |
| Val³⁷⁷ > Ala | | | | |
| Ser⁴¹⁵ > Gly | | | | |
| | | | | |
| pMA111 | 123 | 19.8 | 190 | 10.1 |
| Thr¹⁰⁰ > Val | | | | |
| | | | | |
| pMA112 | 133 | 20.2 | 229 | 10.2 |
| Thr¹⁰⁰ > Ile | | | | |
| | | | | |
| pMA113 | 220 | 94.4 | 217 | 47.7 |
| Lys³²⁸ > Arg | | | | |

The thermal stability of the enzymes was measured according to the following procedure. The time course of irreversible thermoinactivation of each alkaline phosphatase was measured by incubating a solution of the enzyme (20 µg/ml, prepared in 0.02 M Tris-HCI, pH 7.5 at heating temperature, containing 1 mM MgCl₂) in a constant temperature heating block and periodically removing samples to assay them at 25°C. First order rate constants and half-lives for irreversible thermoinactivation were determined by linear regression in semilogarithmic coordinates, and correlation coefficients of at least 0.97 were obtained in all cases. Examples of the time courses of irreversible thermoinactivation for several of the alkaline phosphatases, at 85°C and pH 7.5, are shown in Table 5.

**Table 5**

| Time Courses of Irreversible Thermoinactivation | | | |
|---|---|---|---|
| Time | % Residual Activity | | |
| (min) | pMA100 | pMA111 | pMA112 |
| 0 | 100 | 100 | 100 |
| 3 | 102 | 91 | 78 |
| 6 | 102 | 82 | 60 |
| 9 | 96 | 76 | 53 |
| 12 | 97 | 69 | 44 |
| 15 | 96 | 63 | 39 |

The results of similar studies for each the mutant enzymes are presented in Table 6. The mutant enzymes all underwent an irreversible thermoinactivation at a faster rate than did the native alkaline phosphatase. The thermostability of the mutant enzymes, however, remained superior to the thermostability of the calf-intestinal alkaline phosphatase which has a half-life of six minutes at only 70°C under similar assay conditions.

**Table 6**

| Thermostability of the Mutant Enzymes | | | |
|---|---|---|---|
| Alkaline Phosphatase | Temperature (°C) | Rate (min.⁻¹) | Half-Life (min.) |
| pMA100 | 95 | 0.126 | 5.5 |
| wild-type | | | |
| | | | |
| pMA110 | 80 | 0.100 | 6.9 |
| Val³⁷⁷ > Ala | | | |
| Ser⁴¹⁵ > Gly | | | |
| | | | |
| pMA111 | 85 | 0.031 | 22.6 |
| Thr¹⁰⁰ > Val | | | |
| | | | |
| pMA112 | 85 | 0.062 | 11.1 |
| Thr¹⁰⁰ > lie | | | |
| | | | |
| pMA113 | 85 | 0.064 | 10.8 |
| Lys³²⁸ > Arg | | | |
| | | | |
| pMA114 | 80 | 0.040 | 17.3 |
| Val⁹⁹ > Ala | | | |
| | | | |
| pMA115 | 80 | 0.051 | 13.6 |
| Asp¹⁰¹ > Ser | | | |
| | | | |
| pMA116 | 75 | 0.138 | 5.0 |
| Lys³²⁸ > Arg | | | |
| Val⁹⁹ > Ala | | | |

### Example 6

### Effect Of pH On The Kinetic Constants Of Alkaline Phosphatases

The effects of pH on the kinetic constants (Kₘ and Vₘₐₓ) were determined for both the mutant enzyme pMA115 (Asp¹⁰¹ > Ser) and calf-intestinal alkaline phosphatase (Boehringer Mannheim, Indianapolis, IN). The measurements were made at 25°C using 4-MUP as the substrate. The release of methylumbelliferone from the substrate was monitored by following the increase in absorption at 360 nm (extinction coefficients were experimentally determined using methylumbelliferone and are shown in Table 7) using a Hewlett-Packard 9153A UV Spectrophotometer. Low ionic strength buffers (I=200) containing 50 mM Tris (pH 8-9.5) or 50 mM diethanolamine (Sigma) (pH 9-11) and NaCI (Sigma) were used. Initial rates were determined graphically from the first 5-10% of the reaction(r > 0.995). Values of Vₘₐₓ and Kₘ were obtained from the Lineweaver-Burk plots of six to seven points. The conversion of Vₘₐₓ to k_{cat} was performed using molecular weights of 150,000 daltons and 94,000 daltons for calf-intestinal and pMA115 alkaline phosphatases, respectively, as well as using two active sites per dimer for both enzymes. The results are shown in Table 7.

**Table 7**

| Effect of pH on the Kinetic Constants of Alkaline Phosphatases Using 4-MUP as a Substrate | | | | | | | |
|---|---|---|---|---|---|---|---|
| | | Alkaline Phosphatase | | | | | |
| pH | Extinction Coefficient | BMA115 | | | Calf-intestinal | | |
| | (M⁻¹cm⁻¹) | Kₘ (µM) | Vₘₐₓ (µM/min) | k_{cat} (sec⁻¹) | Kₘ (µM) | Vₘₐₓ (µM/min) | k_{cat} (sec⁻¹) |
| 8.0 | 1.09 x 10⁴ | 3.1 | 11.4 | 30 | 7.9 | 48.4 | 303 |
| 8.5 | 1.4 x 10⁴ | 6.7 | 26.5 | 69 | 18.2 | 58.7 | 367 |
| 9.0 | 1.62 x 10⁴ | 50.3 | 66.2 | 173 | 43.9 | 91.9 | 574 |
| 9.5 | 1.65 x 10⁴ | 410 | 176 | 459 | 111 | 140 | 875 |
| 10.0 | 1.67 x 10⁴ | 1660 | 262 | 684 | 348 | 161 | 1130 |
| 10.5 | 1.68 x 10⁴ | 3520 | 284 | 741 | 4762 | 632 | 3950 |
| 11.0 | 1.69 x 10⁴ | 3630 | 239 | 623 | 16600 | 1116 | 6980 |

The values of Kₘ and k_{cat} Increase with pH for both enzymes over nearly the entire pH range studied. The smallest difference in k_{cat} between the mutant *E. coli* and calf-intestinal alkaline phosphatase exists at pH 10.0. At this pH, the calf-intestinal enzyme is only 1.65 times faster than the mutant *E. coli* enzyme. Even with the increased Kₘ value at pH 10.0, the novel enzymes are suitable for use as labels in assays. Similar experiments were conducted for these enzymes, as well as for the native *E. coli* alkaline phosphatase (pMA100), using pNPP as the substrate. The conversion of the pNPP substrate to p-nitrophenol was monitored by following the change in absorption at 410 nm (e = 1.62 x 10⁴ M ^{⁻ 1} cm⁻¹) using the Hewlett-Packard 9153A UV Spectrophotometer. The effect of pH on k_{cat} for these enzymes is shown in Table 8. The k_{cat} for the enzyme PMA115 was nearly 36 times higher than that of the wild type *E. coli* alkaline phosphatase at pH 10.0.

**Table 8**

| Effect of pH on k_{cat} of Alkaline Phosphatases Using pNPP as a Substrate | | | |
|---|---|---|---|
| | k_{cat} for Alkaline Phosphatases | | |
| pH | pMA100 | pMA115 | Calf-Intestinal |
| 8.0 | 8 | 35 | 345 |
| 8.5 | 10 | 78 | 480 |
| 9.0 | 12 | 154 | 760 |
| 9.5 | 13 | 255 | 1004 |
| 10.0 | 30 | 1068 | 2113 |
| 10.5 | 33 | 947 | 4675 |
| 11.0 | 28 | 927 | 7175 |

The trends observed for pNPP are similar to those obtained with 4-MUP: k_{cat} increases from pH 8 to 10, and the minimal difference in k_{cat} between pMA115 and the calf-intestinal alkaline phosphatase exists at pH 10.0 (2-fold difference exists). A large difference between the k_{cat} for native *E. coli* (pMA100) and calf-intestinal alkaline phosphatase exists throughout the pH range where, even at pH 10.0, there is a 70-fold difference in k_{cat}. The data clearly demonstrates that pMA115 is superior to the wild type *E. coli* alkaline phosphatase because, in a comparison to the calf-intestinal enzyme, the difference in k_{cat} at pH 10.0 is reduced from 70-fold to a mere 2-fold.

### Example 7

### Use Of Mutant Alkaline Phosphatase In A Sandwich EIA For Alpha-Fetoprotein

An enzyme labeled anti-AFP antibody was prepared by first treating the mutant enzyme (alkaline phosphatase from pMA113, prepared as described in Example 3) and the monoclonal anti-AFP antibody separately, as follows. A 50-fold molar excess of m-maleimidobenzoyl-N-hydroxysuccinimide ester (SMCC) in N,N-dimethylformamide (DMF) was added to an 0.6 mg/ml solution of alkaline phosphatase in 0.1 M phosphate buffer (pH 7.2, containing 1 mM MgCl₂; with a final DMF concentration of 5%). The reaction was carried out for three hours at 25°C, after which the SMCC treated enzyme was dialysed against an 0.1 M phosphate buffer (pH 7.2, containing 1.0 mM MgCl₂) at 4°C for 18 hours.

A 5.8 mg/ml anti-AFP antibody solution in 0.1 M phosphate (pH 72, containing 1.0 mM MgCl₂) was treated with a 500-fold molar excess of 2-iminothiolane for one hour at 25°C. The thiolated sample was then dialysed for 18 hours at 4°C against an 0.1 M phosphate buffer (pH 7.2, containing 1 mM MgCl₂).

The SMCC treated enzyme was added to the thiolated anti-AFP antibody at a 2 :1 (respectively) molar ratio and a one milligram/milliliter total protein concentration. The reaction was stopped after four hours at 4°C by the addition of N-ethylmaleimide (final concentration of 0.3 mM) for 30 minutes at 4°C, followed by a 2-mercaptoethanol addition (final concentration of 1.0 mM), again for 30 minutes at 4°C. The solution was then dialysed for 18 hours at 4°C against a 20 mM Tris-HCI buffer (pH 8.0, containing 1.0 mM MgCl₂).

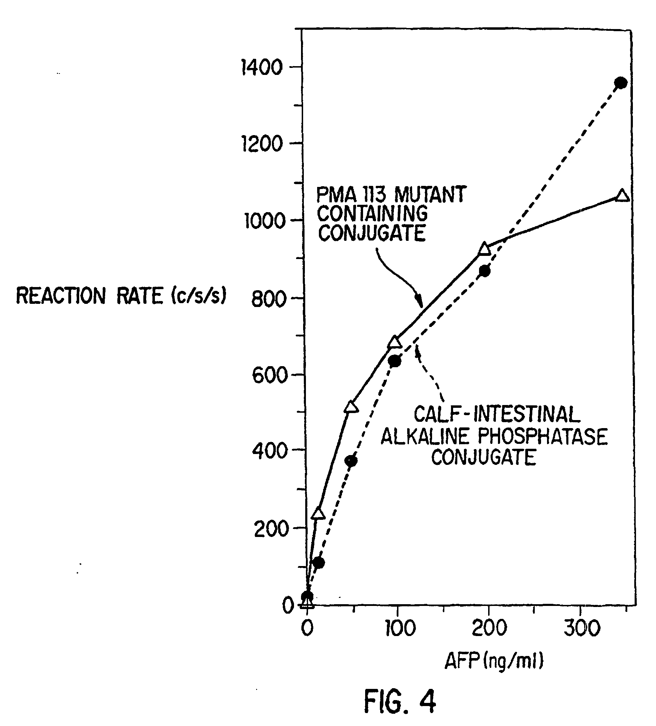

The performance of the enzyme/antibody indicator reagent was evaluated using the Abbott IMx®-AFP Assay protocol and reagents (Abbott Laboratories, Abbott Park, IL). The substrate for the *E. coli* mutant alkaline phosphatase indicator reagents contained 1.2 mM 4-MUP in 1.5 M Tris-HCI buffer (at pH 8.0, containing 1 mM MgCl₂). The enzyme/antibody indicator reagent was diluted to a final concentration of 1.3 micrograms/milliliter with indicator reagent diluent buffer from the assay kit and was then filtered (0.2 µM membrane). The indicator reagent was used to generate the standard curve shown in Figure 4. Also included in Figure 4 is the standard curve obtained using a calf-intestinal alkaline phosphatase-labeled anti-AFP antibody indicator reagent. The correlation between the curves demonstrated that the mutant enzyme/antibody indicator reagents can be used in an assay at concentrations as low as 1.3 micrograms/milliliter as compared to the mammalian enzyme/antibody indicator reagent used at approximately 0.8 micrograms/milliliter.

### Example 8

### Use Of Mutant Alkaline Phosphatase In A Sandwich EIA For Cancer Antigen

An anti-cancer antigen antibody fragment was labeled with a mutant alkaline phosphatase according to the following procedure. A solution of mutant alkaline phosphatase from pMA115 (0.6 mg/ml, in an 0.1 M phosphate buffer, pH 7.2, containing 1.0 mM MgCl₂) was treated with a 450-fold molar excess of 2-iminothiolane for 30 minutes at 25°C with gentle rotation. The thiolated sample was desalted on a Sephadex G-25 column (1 x 45 cm) equilibrated and eluted with a 0.1 M phosphate buffer (pH 7.0, containing 0.1 M NaCl, 1.0 mM MgCl₂ and 0.1 mM ZnCl₂).

The antibody fragment was reacted with a 50-fold molar excess of succinimidyl 4-(N-maleimidomethyl-1-tricapramide) cyclohexane carboxylate (30 atom linker), prepared in DMF (with a final DMF concentration of 15%) for 30 minutes at 25°C with gentle rotation. This sample was then desalted on a Sephadex G-25 column (1 x 45 cm) equilibrated and eluted with a 0.1 M phosphate buffer (pH 7.0, containing 0.1 M NaCI and 5 mM EDTA).

The thiolated mutant alkaline phosphatase was mixed with the activated antibody fragment at a molar ratio of 1.5:1, respectively. The reaction mixture was gently rotated for 15 hours at 2-8°C, and the reaction was terminated by the addition of N-ethylmaleimide (to a final concentration of 0.1 mM). After one hour at 25°C, the sample was dialysed against a Tris-HCI buffer (20 mM, pH 8.0 containing 1.0 mM MgCl₂) for 18 hours at 4°C.

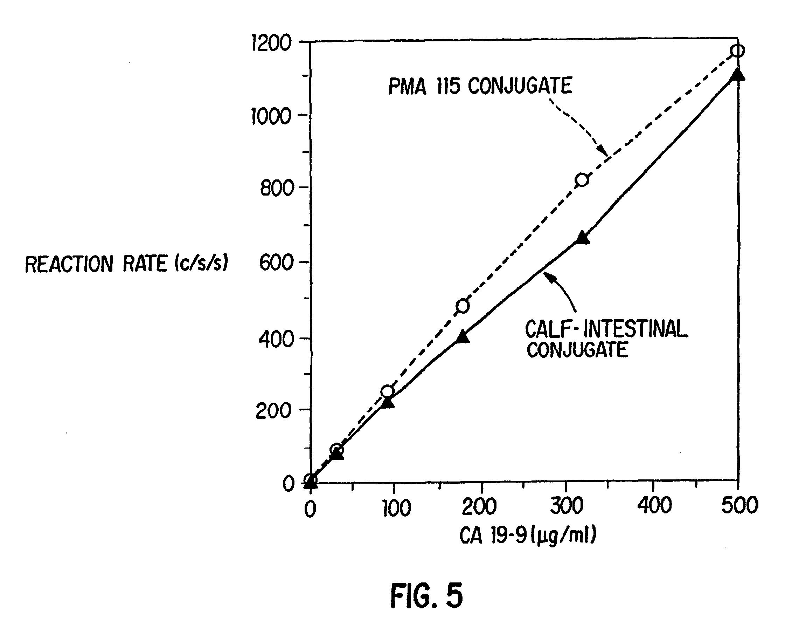

The resultant enzymelantibody fragment indicator reagent was diluted to a final concentration of 3.7 micrograms/milliliter with indicator reagent diluent buffer and was filtered, substantially in accordance with the procedure described in Example 7. The indicator reagent was used, substantially in accordance with the assay procedure described in Example 7, in a sandwich assay to detect cancer antigen and to generate the standard curve shown in Figure 5. Also shown in Figure 5 is the standard curve obtained using a similarly prepared calf-intestinal alkaline phosphatase/antibody fragment indicator reagent at an assay concentration of 1.64 micrograms/milliliter. Considering the difference in indicator reagent concentration, the performance of the mutant enzyme/antibody fragment indicator reagent is comparable to that of the mammalian enzyme/antibody fragment indicator reagent. The assay results demonstrated that an indicator reagent which uses the mutant enzyme as a label can be used successfully in an EIA.

### Example 9

### The Stability Of Mutant Alkaline Phosphatase/Binding Member Indicator Reagents

An enzyme-labeled anti-AFP antibody was prepared substantially in accordance with the procedure described in Example 7, with the following exceptions: (1) a 0.6 mg/ml solution of mutant alkaline phosphatase from pMA110 (in 0.1 M phosphate buffer) was treated for two hours at 25°C with a 50-fold molar excess of 30-atom linker dissolved in DMF (with a final DMF concentration of 15%) and (2) the activated enzyme and a thiolated antibody (prepared substantially in accordance with the procedure described in Example 7) were reacted at a 1:1 molar ratio for eight hours at 4°C. The resultant indicator reagent was diluted to 15 micrograms/milliliter with indicator reagent diluent buffer (as described in Example 6) and thermally stressed at 45°C.

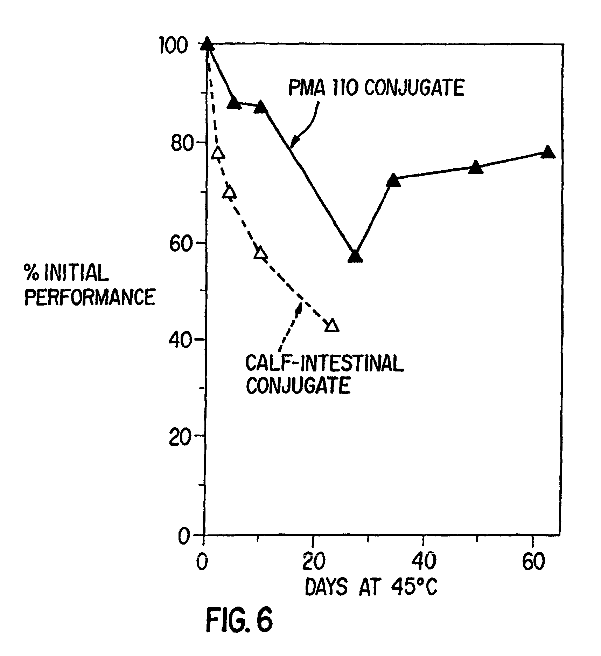

To follow the time course of the indicator reagent's thermal inactivation, samples were removed from the heat over a period of 60 days, and the performance of the indicator reagent was measured in accordance with the protocol described in Example 6. The assay results are presented in Figure 6 which also illustrates the time course of thermal inactivation of a calf-intestinal alkaline phosphatase/anti-AFP antibody indicator reagent. The indicator reagent containing the *E. coli* mutant alkaline phosphatase lost less than 30% of its initial signal after 60 days at 45°C, whereas the mammalian enzyme/antibody indicator reagent lost greater than 60% of its initial signal in only 20 days at 45°C. The thermal stability of the *E. coli* mutant enzyme/antibody indicator reagent was far superior to that of the mammalian enzyme indicator reagents.

### Example 10

### Site-Directed Conjugation Of Mutant Alkaline Phosphatase And Specific Binding Members

This experiment involved the preparation of a mutant *E. coli* alkaline phosphatase exhibiting a high specific activity and containing one surface cysteine residue per monomer. The presence of the reactive cysteine residue enabled the site-specific covalent linkage of the mutant enzyme to a specific binding member via a heterobifunctional crosslinking reagent. This linking procedure eliminated the need to introduce thiols to the enzyme via the nonspecific chemical modification, using 2-iminothiolane, as described in Examples 7 and 8. The resultant specific binding member/enzyme conjugates have enhanced stability and a lower incidence of nonspecific binding in an EIA.

The enzyme was prepared substantially in accordance with the procedure described in Example 3, and the cysteine residue was incorporated into the enzyme molecule, substantially in accordance with the procedure described in Example 3, at a location which did not interfere with either the active site of the enzyme or the monomer interface. The modified enzyme was then conjugated to a specific binding member by means of a heterobifunctional crosslinking agent, such as m-maleimido-benzoyl-N-hydroxysuccinimide ester or succinimidyl 4-(maleimidomethyl-1-tricarpamide cyclohexane carboxylate), substantially in accordance with the procedures described in Examples 7 and 8.

### Example 11

### Construction Of A Synthetic E. coli Plasmid With Unique Restriction Sites

### a. General

A synthetic *E. coli* plasmid was designed, constructed and shown to be a functional cloning vector. The *Fok*I method of gene synthesis (Mandecki and Boiling, Gene, 68, 101; 1988) was used to assemble the plasmid from 30 oligonucleotides. The plasmid contained synthetic modules for the β-lactamase gene, replication origin, *IacZ* gene fragment and multicloning site, and is patterned after the pUC-type plasmids. The differences include the removal of nearly 50% of the restriction sites present in pUC plasmids, the reduction of plasmid size to 2050 base pairs and the introduction of transcription terminators downstream of both the β-lactamase gene and *lacZ* fragment. These changes facilitate a number of techniques, such as cloning, mutagenesis, expression and restriction analysis.

### b. Design Of The Plasmid

The overall design of the synthetic plasmid of *E. coli* was based upon traits necessary or desirable to a cloning/expression vector. One requirement was a low molecular weight to facilitate manipulations and purification of restriction fragments derived from the vector. Another favorable characteristic was the exclusion of as many restriction sites as possible, while introducing unique restriction sites at critical locations. Past experience has shown that subsequent subcloning of a DNA fragment entails a distinct disadvantage when cloning into a vector with many diverse restriction sites.

The synthetic plasmid was divided into three separate cassettes. First, the origin of replication from pUC9 [Vieira and Messing, Gene, Volume 19, pages 259-268 (1982)] was chosen as the DNA sequence which would constitute the *ori* region in the synthetic plasmid. The sequence contains both the RNA I and RNA II replication primer regions, as well as their respective promoters (Polisky, Maximizing Gene Expression, W.S. Reznikoff, Ed., Butterworths, Boston, 1986).

Second, the β-lactamase gene of the pUC plasmids was chosen as a selection marker. The gene included the natural P3 promoter [Brosius, et al., J. Biol. Chem., Volume 257, pages 9205-9210 (1982)] found in pUC9 and the strong phage fd gene *VIII* transcription terminator [Beck, et al., Nucleic Acids Res., Volume 5, pages 4494-4510 (1978)]. In contrast to the *ori* region, the nucleotide sequence for β-lactamase was changed to remove several restriction sites. In most cases (except Ile⁸² > Val and Val¹⁸² > Ala) the amino acid sequence remained the same. Approximately 60% of the naturally occurring restriction sites in the *bla* gene were removed.

Third, the a-complementing *lacZ* gene fragment of pUC was also desirable because of its usefulness as a cloning marker and its expression of heterologous fusion proteins, in addition to having an *ori* region and ampicillin resistance gene. The *lacZ* sequence from pUC9 was changed to reduce the number of restriction sites analogous to the changes in the β-lactamase gene. The *Sma*l site was maintained as a unique restriction site for the insertion of any other desired site(s).

### c. Genetic Constructions

A total of 25 oligonucleotides were synthesized for the construction of pWM510 using the *Fok*l method of gene synthesis, and the oligonucleotides were cloned into the pWM500 series of plasmids [Mandecki and Bolling, Gene, Volume 68, pages 101-107 (1988)] as described above. The plasmids were purified and sequence verified prior to excision of the individual fragments by cutting with *Fok*l. All 25 fragments (ranging in size from 40 base pairs to 82 base pairs) contained unique complementary four base pair overhangs which, when annealed and ligated, formed a complete dosed circular vector. The fragments were ligated and transformed into SCS-1 competent cells [F⁻, *recAl, endAI, gyrA96, thi, hsdR17*, (rₖ⁻, mₖ⁺), *supE44, relAl* l⁻]. Transformed cells were plated on LB plates containing ampicillin. Successfully transformed cells could survive and form colonies only if they carried the intact plasmid containing functional origin of replication and β-lactamase genes.

Ligation was performed using a shotgun ligation of all 25 *Fok*I fragments. Approximately 38 transformants per ten milligrams of ligation mix were obtained (overall transformation efficiency for SCS-1 cells was greater than 5 x 10⁷ cells/mg supercoiled pBR322 [as described in Bolivar, et al., Gene, Volume 2, pages 95-113 (1977)].

A total of three colonies were picked from the plates. Of the three clones, two had correct Avail restriction patterns. A clone was picked, and plasmid DNA was isolated on a CsCl gradient for sequencing. Multiple sequencing primers were used for double-stranded DNA sequence verification. Out of a total of 1659 base pairs, no sequence errors were detected in individual *Fok*I clones or in the assembled plasmid. The synthetic plasmid which contained the *ori* region and a functional β-lactamase gene was designated pWM510.

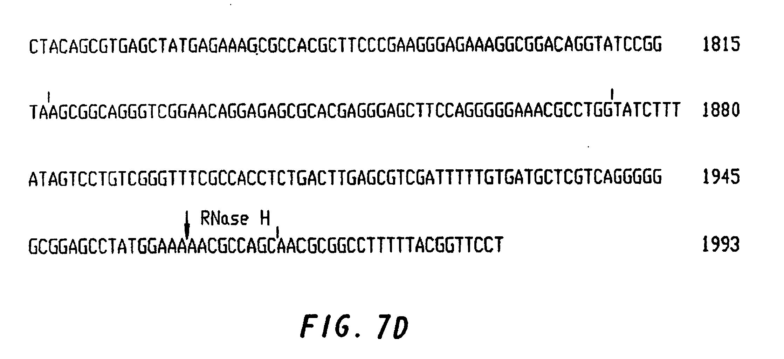

In the second stage of plasmid engineering, the synthetic *lacZ* cassette [as described in Yanish-Perron, et al., Gene, Volume 33, pages 103-119 (1985)] was cloned into the *Eco*RI site of pWM510, using the *Fok*I fragments presented in Figure 2. The cassette comprised the *lac* promoter, the *lacZ* gene fragment encoding 60 amino-terminal amino acids of β-galactosidase, the *trpA* transcription terminator [Christie, et al., Proc. Natl. Acad. Sci. USA, Volume 78, pages 4180-4184 (1981)] and the *Sma*l site for the introduction of a multiple cloning site by bridge mutagenesis. The cloning yielded plasmid pWM511. Although the *lacZ* cassette could be ligated to *Eco*RI-cleaved pWM510 in either of two orientations, only clones expressing the *lacZ* transcription unit in the same direction as β-lactamase mRNA or RNA II were recovered (from 20 clones tested). The orientation of the *lacZ* gene fragment in pWM511 is therefore the same as in pUC-type plasmids. The unique *Fok*I site located in the β-lactamase gene was also removed to enable the use of the synthetic plasmid as a cloning vector for the *Fok*I method of gene synthesis. Because this particular method depends upon the principle of cutting out small gene fragments from a plasmid with *Fok*I, the absence of *Fok*I sites elsewhere in the plasmid greatly facilitates the purification of the small *Fok*I fragments. The removal of the *Fok*I site was accomplished through bridge mutagenesis using a degenerate oligonucleotide to alter the Trp-Met amino acid sequence. Subsequent sequencing showed that the sequence was changed to a Trp-Leu amino add sequence. The Trp-Met sequence corresponds to the DNA region at residues 1060 to 1064 (Figure 8). The sequence of the oligonucleotide used for mutagenesis was: 
GGCAACAATTAATAGACTGGNNNGAAGCGGATAAAGTTGCAGGACCAC
TACT
 The pWM511 plasmid was linearized with *Fok*I, and the bridge mutagenesis method was used to introduce the sequence change. This conservative amino acid mutation caused no observable change in ampicillin resistance. The plasmid construct without the *Fok*I site was named pWM515.

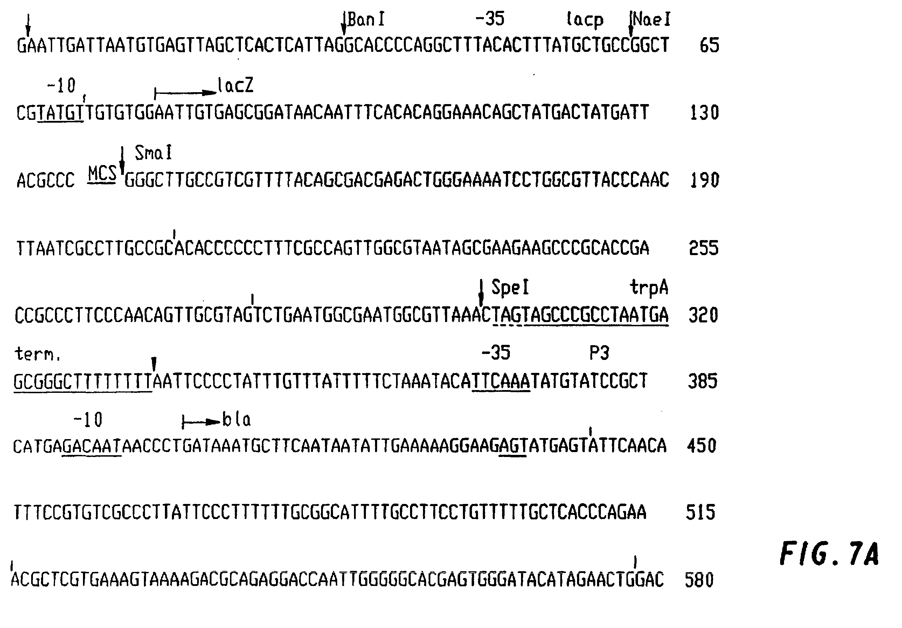

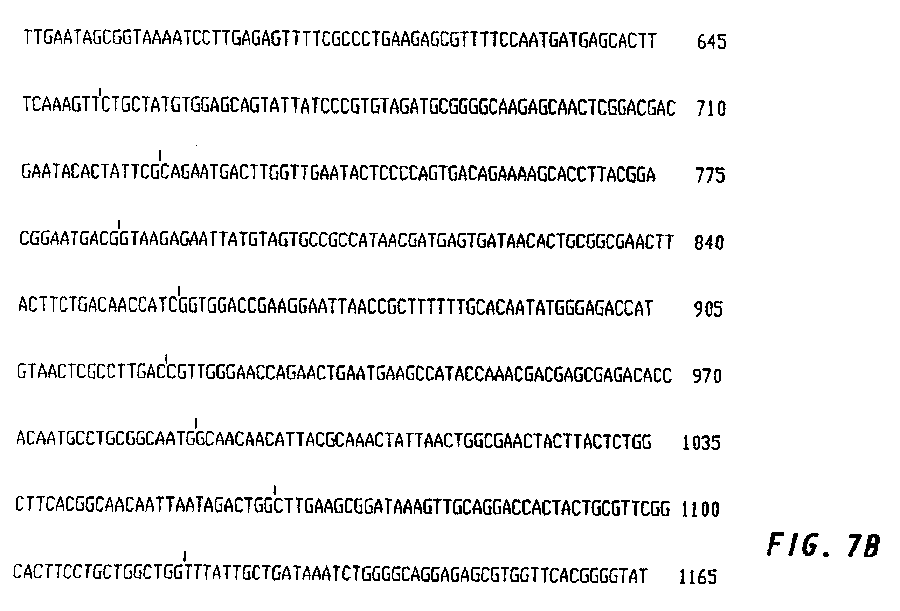

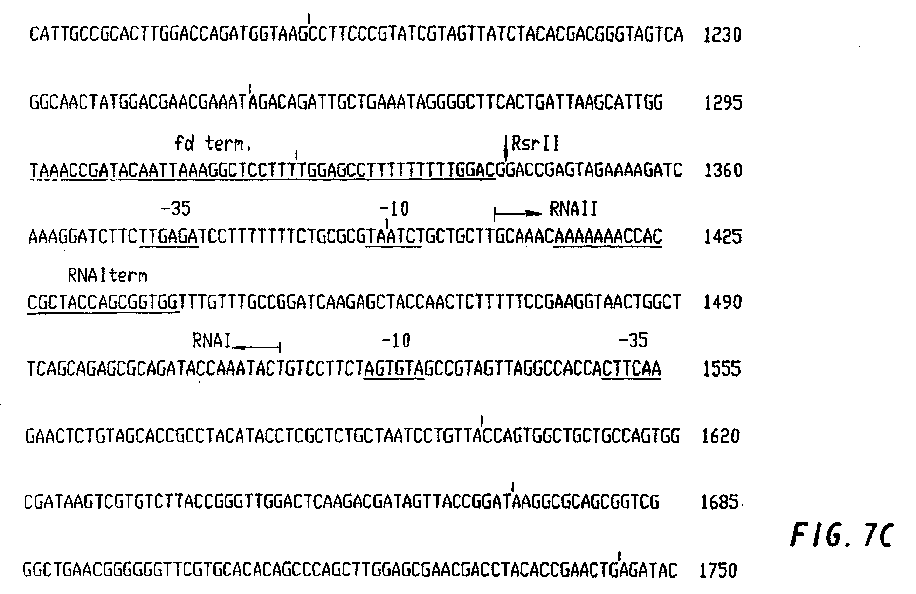

Deletion of the *Eco*RI region was achieved by cutting pWM515 with *Eco*Rl/*Sma*l and incorporating a synthetic duplex oligonucleotide containing the necessary base changes. The synthetic plasmid construct was designated pWM520 (see Figure 7). Bridge mutagenesis was used to clone the multiple cloning sites from phage M13 mp18 [as described in Yanish-Perron, et al., Gene, Volume 33, pages 103-119 (1985)] into the *Sma*I site within the *IacZ* gene. This was done to accommodate standard cloning protocols established for pUC18. The construct containing the multiple cloning site mp18 was named pWM518. The multiple cloning site in pWM518 follows:

GAATTCGAGCTCGGTACCCGGGGATCCTCTAGAGTCGACCTGCAGGCA
TGCAAGCTT.

### d. Characterization Of The Plasmid

Characterization of the synthetic plasmid was of interest because it constituted the first totally synthetic replicon. It is significantly different from its prototype, the pUC-type plasmid. The synthetic plasmid, when compared to pUC-type plasmids, contained three deletions, the total length of which is 636 base pairs, as well as 70 point mutations. Nearly 50% of the restriction sites present in the pUC plasmids were removed, which is advantageous for restriction analysis or purification of DNA. in particular, the pWM519 plasmid contains only seven sites (including three sites incorporated by design to facilitate manipulations) for restriction enzymes recognizing six base pair nondegenerate sequences. There are 24 such sites in the pUC plasmid, not including the multiple cloning site. The low number of such sites allows for the use of virtually any restriction enzyme having a six base pair specificity for cloning, as well as for the mutagenesis of cloned genes by restriction fragment replacement with synthetic DNA or bridge mutagenesis. Plasmid pWM520 does not have cleavage sites for 75 restriction enzymes. The synthetic plasmid is missing the P1 promoter for the β-lactamase gene. It contains the minimum length replication origin, two newly introduced transcription terminators and the minimum length engineered *lac* promoter.

It was shown that the plasmid can be stably propagated for at least 120 generations (four passages on plates). Thus, the constructed fragment containing the replication origin (1349 - 1993 in Figure 7) was fully capable of sustaining stable replication. The plasmid copy number was evaluated by scanning the density of plasmid DNA bands on an agarose gel, measuring the yield of DNA from large DNA preparations and by assaying levels of β-lactamase [Jones, et al., J. Clinic, Microbiol., Volume 15, pages 677-683 (1982)]. The copy number was found to be 3-4 times lower than that of pUC9, and equivalent to the pBR322 copy number. This observation was consistent with the recently determined sequence change in the PUC-type plasmid origin of replication compared to pBR322 [G at 2990 in pBR322 changed to an A in a corresponding region of pUC plasmids; Milton, et al., Focus, BRL-Gibco Volume 10, page 56 (1988)], which seems to be conferring a higher copy number to pUC plasmids.

The sequence of the synthetic plasmid is presented in Figure 7. Transcription terminators, -35 and -10 promoter regions, and fMet-encoding ATG triplets are underlined in Figure 7. Horizontal arrows indicate transcription start sites. A vertical arrow indicates the RNaseH cleavage site. Apostrophes indicate the division points and give the sequences of *Fok*I fragments. The sequences of synthetic oligonucleotides were: arm 1 + sequence between division points + overlap of four 3' terminal residues + arm 2. The *Fok*I fragments and oligonucleotides are numbered as indicated above the sequence. The sequence of fragment one is composed of two discontinuous sequences. Triangles define the *lacZ* cassette.

All oligonucleotides used for construction of pWM510 were synthesized on an Applied Biosystems 380A Synthesizer using 5'-dimethoxytrityl nucleoside β-cyanoethyl phosphoramidites. Syntheses were carried out on 0.2 micromolar scale controlled pore glass solid support with an average pore size of 1000 Å. Oligonucleotides were purified by gel electrophoresis.

Cloning of the synthetic oligonucleotides was accomplished by the bridge mutagenesis protocol. All four cloning vectors (pWM500, pWM501, pWM502 and pWM507) used for the *Fok*I method of gene synthesis were cut with *Sma*l. Approximately 50 nanograms of linearized vector was mixed with 20 picomoles of oligonucleotide in 30 microliters of denaturation buffer (10 mM KCI, 5.0 mM Tris-HCl pH 8.0, 5.0 mM MgSO₄, 0.5 mM dithiothreitol), and the mixture was heated at 100°C for three minutes in a boiling water bath. The samples were cooled to room temperature for five minutes and transferred to 200 milliliters of chilled competent JM83 cells (*ara*, D(*lac-proAB*), *strA, thi,* F80 *lacz*D*M15*). Competent cells were prepared by the CaCl₂ procedure. The DNA/cell mixture was chilled on ice for five minutes followed by a three minute heat shock at 37°C. Approximately two microliters of LB media was added to the transformation mix, the cells were incubated at 37°C for one hour, and then the cells were plated.

Plasmid constructs containing the *Fok*I fragments for the synthetic plasmid were digested as follows. Approximately 200 nanograms of each plasmid was cut with 90 units of *Fok*I (New England BioLabs, Beverly, MA). Reactions were carried out in 500 microliter volumes containing 1x *Fok*I buffer (20 mM KCI, 10 mM Tris-HCl pH 7.5, 1.0 mM MgCl₂ and 10 mM 2-mercaptoethanol) at 37°C for 2.5 hours. The insert-containing *Fok*I fragments were then purified by polyacrylamide gel electrophoresis.

In the ligation procedure, all 25 *Fok*I fragments (100 ng each) were joined together in a single reaction (see above). The types of cloning vectors used were as follows: pWM500 - for fragments 2 to 14 and 26 to 29; pWM501 - for fragments 15 to 18, 24 and 25; pWM502 - for fragments 19 to 23, and pWM507 - for fragment 30.

### Example 12

### Construction And Properties Of Asp¹⁵³>Gly Mutant

An Asp¹⁵³>Gly mutant was constructed by introducing a mutation into plasmid pMA101 by first cutting it with Spel and Pvull restriction enzymes to release the DNA fragment carrying the native codon for amino acid residue 153. The cleaved DNA was then used in bridge mutagenesis as described in Example 1(b) together with an oligonucleotide that carried a random DNA sequence at the position corresponding to the desired mutation. The sequence of the nucleotide was:

AAATGGCAAAAGCAGCTGGTCTGGCGACCGGTAACGTTTCTACCGCAGAGTTGCAGNNNG
CCACACCCGCTGCGCTGGTGGCACATGTGACTAGTCGCAAATGCT.

The resulting colonies were screened for high k_{cat} using the chromogenic substrate 5-bromo-4-chloro-3-indolyl-phosphate as follows:
1. After *E. Coli* cells were transformed with the plasmid containing the Asp ¹⁵³>Gly mutation as described above, they were plated onto filters (Millipore No. HATF 082 25) which were placed on LB agar plates containing ampicillin, and the cells incubated overnight at 37°C with the plates covered.
2. Replicas of the plated filters from step 1) were made by pressing them onto fresh filters (Millipore No. HATF 082 25) between two heavy books. The cells on the original filters and the replica filters were placed in separate LB agar plates and incubated for 2-3 hours at 37°C with the plates covered.
3. The plates from step 2) were incubated uncovered at 90°C for 1 hour and 45 minutes, and then allowed to cool to room temperature.
4. The original filters and the replica filters from the incubated plates from step 3) were carefully peeled from the agar and placed on a piece of Whatman blotting paper which was previously wetted with 40 mL of 50mM Tris (pH 10.0) and then incubated for 5 minutes at room temperature.
5. The original filters and the replica filters from step 4) were carefully blotted and then placed on another sheet of blotting paper which was previously wetted with 40 mL 50 mM Tris, 0.2 mg/mL 5-bromo-4-chloro-3-indolyl-phosphate (pH 10.0), and incubated for 5 minutes at room temperature.
6. The original filters and the replica filters from step 5) were carefully moved to a dry sheet of Whatman blotting paper and colonies having the most intense blue color on the original filters were matched to the corresponding colonies on the replica filters.
7. The colonies from the origianl filter from step 6) were removed and cultures grown overnight in 4.0 mL LB broth with ampicillin at 37°C.
8. LB agar plates were divided into quarters and samples from the grown cultures of step 7) were streaked onto the agar plates and incubated overnight at 37°C.

A clone was identified from the screening procedure that exhibited clearly very dark blue color on both the original filter and the replica filter, as compared to the wild-type. The clone, pDG201, was characterized by DNA sequencing, which yielded the sequence GGC for the codon 153, instead of GAC (wild-type sequence). This codon coresponds to the glycine residue at position 153 in the mutant The mutant gene in plasmid pDG201 was subsequently expressed in E.coli, and the mutant alkaline phosphatase purified as described in Example 4. The purified mutant protein was characterized by measuring k_{cat}, its pH dependence, and Km as described in Example 5. In 1M Tris, pH 8.2, the k_{cat} of the mutant was 229 sec⁻¹, and Kₘ was 11.8 uM. The results showed that the Asp 153>Gly mutant is almost as catalytically active as mutant Asp101>Ser over the whole pH range investigated, and therefore is an excellent candidate for several applications, including its use as a reporter enzyme in assays as described herein.

## Claims

1. A synthetic enzyme which is a mutant of an alkaline phosphatase enzyme produced by *Escherichia coli,* wherein said mutant alkaline phosphatase has at least one amino acid mutation as compared to wild-type *Escherichia coli* alkaline phosphatase, **characterized in that** said mutation consists of Gly in place of Asp¹⁵³.

2. An engineered DNA sequence comprising a nucleotide sequence encoding a mutant alkaline phosphatase enzyme for expression in a unicellular host, wherein said alkaline phosphatase has at least one amino acid mutation as compared to the corresponding wild-type *Escherichia coli* alkaline phosphatase, and wherein said mutant alkaline phosphatase has an increased specific activity as compared to wild-type *Escherichia coli* alkaline phosphatase, **characterized in that** said mutation consists of Gly in place of Asp¹⁵³.

3. A plasmid comprising an engineered DNA sequence encoding a mutant alkaline phosphatase enzyme for expression in a unicellular host, wherein said alkaline phosphatase has at least one amino acid mutation as compared to the corresponding wild-type *Escherichia coli* alkaline phosphatase, and wherein said mutant alkaline phosphatase has an increased specific activity as compared to wild-type *Escherichia coli* alkaline phosphatase, **characterized in that** said mutation consists of Gly in place of Asp¹⁵³.

4. A unicellular host, comprising a plasmid comprising an engineered DNA sequence encoding a mutant alkaline phosphatase enzyme for expression in a unicellular host, wherein said alkaline phosphatase has at least one amino acid mutation as compared to the corresponding wild-type *Escherichia coli* alkaline phosphatase, and wherein said mutant alkaline phosphatase has an increased specific activity as compared to wild-type *Escherichia coli* alkaline phosphatase, **characterized in that** said mutation consists of Gly in place of Asp¹⁵³.

5. A method for determining the presence or amount of an analyte in a test sample, comprising the steps of:
a. contacting the test sample sequentially or simultaneously with an indicator reagent and a capture binding member, said indicator reagent comprising an alkaline phosphatase enzyme according to Claim 1 directly or indirectly attached to a specific binding member,
b. allowing said indicator reagent to bind to a member selected from the group consisting of the analyte, said capture binding member and an ancillary specific binding member, thereby forming an indicator reagent complex; and
c. reacting said indicator reagent complex or free indicator reagent with an enzyme substrate to produce a detectable signal and thereby determine the presence or amount of analyte in the test sample.

6. An indicator reagent, useful for determining the presence or amount of an analyte in a test sample, comprising:
a. the mutant alkaline phosphatase enzyme of Claim 1 and
b. a specific binding member directly or indirectly attached to said enzyme.

## Patentansprüche

1. Ein synthetisches Enzym, welches ein Mutant eines alkalischen Phosphatase-Enzymes ist, das durch *Escherichia coli* produziert wird, wobei der alkalische Phosphatase-Mutant mindestens eine Aminosäure-Mutation besitzt, wenn verglichen mit der wild vorkommenden alkalischen Phosphatase von *Escherichia coli*, **dadurch gekennzeichnet, dass** diese Mutation aus Gly anstelle von Asp¹⁵³ besteht.

2. Eine veränderte DNA Sequenz, welche eine Nukleotid-Sequenz umfasst, die einen alkalische Phosphatase-Enzym-Mutanten zur Expression in einem einzelligen Wirt kodiert, worin die alkalische Phosphatase mindestens eine Aminosäure-Mutation besitzt, wenn verglichen mit der entsprechenden wild-vorkommenden *Escherichia coli* alkalischen Phosphatase, und worin der alkalische Phosphatase-Mutant eine erhöhte spezifische Wirksamkeit besitzt, wenn verglichen mit der wild-vorkommenden *Escherichia coli* alkalischen Phosphatase, **dadurch gekennzeichnet, dass** die Mutation aus Gly anstelle von Asp¹⁵³ besteht.

3. Ein Plasmid, welches eine veränderte DNA Sequenz umfasst, die einen alkalische Phosphatase-Enzym-Mutanten zur Expression in einem einzelligen Wirt kodiert, worin die alkalische Phosphatase mindestens eine Aminosäure-Mutation besitzt, wenn verglichen mit der entsprechenden wild-vorkommenden *Escherichia coli* alkalischen Phosphatäse, und worin der alkalische Phosphatase-Mutant eine erhöhte spezifische Wirksamkeit besitzt, wenn verglichen mit wild-vorkommender *Escherichia coli* alkalischer Phosphatase, **dadurch gekennzeichnet, dass** die Mutation aus Gly anstelle von Asp¹⁵³ besteht.

4. Ein einzelliger Wirt, der ein Plasmid umfasst, welches eine veränderte DNA Sequenz umfasst, welche einen alkalische Phosphatase-Enzym-Mutanten zur Expression in einem einzelligen Wirt kodiert, worin die alkalische Phosphatase mindestens eine Aminosäure-Mutation besitzt, wenn verglichen mit der entsprechenden wild-vorkommenden *Escherichia coli* alkalischen Phosphatase, und worin der alkalische Phosphatase-Mutant eine erhöhte spezifische Wirksamkeit besitzt, wenn verglichen mit wild-vorkommender *Escherichia coli* alkalischer Phosphatase, **dadurch gekennzeichnet, dass** die Mutation aus Gly anstelle von Asp¹⁵³ besteht.

5. Ein Verfahren zum Bestimmen der Anwesenheit oder der Menge eines Analyten in einer Testprobe, wobei das Verfahren die folgenden Schritte umfasst:
a. in Kontakt bringen der Testprobe nacheinander oder gleichzeitig mit einem Indikator-Reagenz und einem Einfang-Bindungsglied, wobei das Indikator-Reagenz ein alkalisches Phosphatase-Enzym gemäß Anspruch 1 umfasst, direkt oder indirekt an ein spezifisches Bindungsglied angeheftet,
b. dem Indikator-Reagenz erlauben, an ein Glied zu binden, das gewählt ist aus der Gruppe bestehend aus dem Analyten, dem Einfang-Bindungsglied und einem untergeordneten spezifischen Bindungsglied, wobei ein Indikator-Reagenz-Komplex gebildet wird; und
c. Reagieren des Indikator-Reagenz-Komplexes oder des freien Indikator-Reagenzes mit einem Enzym-Substrat, um ein detektierbares Signal zu produzieren und dabei die Anwesenheit oder die Menge an Analyt in der Testprobe zu bestimmen.

6. Ein Indikator-Reagenz, das nützlich ist zur Bestimmung der Anwesenheit oder der Menge eines Analyten in einer Testprobe, das folgendes umfasst:
a. den alkalische Phosphatase-Enzym-Mutanten von Anspruch 1, und
b. ein spezifisches Bindungsglied, das direkt oder indirekt an das Enzym angeheftet ist.

## Revendications

1. Une enzyme synthétique laquelle est un mutant d'une enzyme phosphatase alcaline produite par *Escherichia coli*, en quoi ladite phosphatase alcaline mutante a au moins une mutation d'acide nucléique par comparaison à la phosphatase alcaline d*'Escherichia coli* de type sauvage, **caractérisée en ce que** ladite mutation consiste en une Gly à la place de l'Asp¹⁵³.

2. Une séquence d'ADN modifiée comprenant une séquence nucléotidique codant une enzyme phosphatase alcaline mutante pour expression dans un hôte unicellulaire, en quoi ladite phosphatase alcaline a au moins une mutation d'acide aminé par comparaison à la phosphatase alcaline d*'Escherichia coli* de type sauvage correspondante, et en quoi ladite phosphatase alcaline mutante a une activité spécifique augmentée par comparaison à la phosphatase alcaline d*'Escherichia coli* de type sauvage, **caractérisée en ce que** ladite mutation consiste en une Gly à la place de l'Asp¹⁵³.

3. Un plasmide comprenant une séquence d'ADN modifiée codant une enzyme phosphatase alcaline mutante pour expression dans un hôte unicellulaire, en quoi ladite phosphatase alcaline a au moins une mutation d'acide aminé par comparaison à la phosphatase alcaline d*'Escherichia coli* de type sauvage correspondante, et en quoi ladite phosphatase alcaline mutante a une activité spécifique augmentée par comparaison à la phosphatase alcaline d*'Escherichia coli* de type sauvage, **caractérisée en ce que** ladite mutation consiste en une Gly à la place de l'Asp¹⁵³.

4. Un hôte unicellulaire, comprenant un plasmide comprenant une séquence d'ADN modifiée codant une enzyme phosphatase alcaline mutante pour expression dans un hôte unicellulaire, en quoi ladite phosphatase alcaline a au moins une mutation d'acide aminé par comparaison à la phosphatase alcaline d*'Escherichia coli* de type sauvage correspondante, et en quoi ladite phosphatase alcaline mutante a une activité spécifique augmentée par comparaison à la phosphatase alcaline d*'Escherichia coli* de type sauvage, **caractérisée en ce que** ladite mutation consiste en une Gly à la place de l'Asp¹⁵³.

5. Une méthode pour déterminer la présence ou la quantité d'une substance dans un échantillon d'essai, comprenant les étapes consistant à :
a. mettre en contact l'échantillon d'essai de manière séquentielle ou simultanée avec un réactif indicateur et un élément de fixation de capture, ledit réactif indicateur comprenant une enzyme phosphatase alcaline selon la Revendication 1 directement ou indirectement attachée à un élément de fixation spécifique,
b. permettre audit réactif indicateur de se lier à un élément sélectionné dans le groupe se composant de la substance, dudit élément de fixation de capture et d'un élément de fixation spécifique auxiliaire, formant ainsi un complexe de réactif indicateur ; et
c. faire réagir ledit complexe de réactif indicateur ou le réactif indicateur libre avec un substrat enzymatique pour produire un signal détectable et ainsi déterminer la présence ou la quantité de substance dans l'échantillon d'essai.

6. Un réactif indicateur, utile pour déterminer la présence ou la quantité d'une substance dans un échantillon d'essai, comprenant :
a. une enzyme phosphatase alcaline mutante selon la Revendication 1 et
b. un élément de fixation spécifique directement ou indirectement attaché à ladite enzyme.
